# EUROPEAN PATENT APPLICATION

(11) **EP 4 067 507 A2**
(43) Date of publication of application: **05.10.2022**
(21) Application number: 21207208.6
(22) Date of filing: 29.10.2015
(51) Int. Cl.: C12Q 1/70, C12Q 1/68, C12Q 1/6888

(54) **PNA PROBES, KITS AND METHODS FOR DETECTING GENOTYPES OF HUMAN PAPILLOMAVIRUS**

(30) Priority: 07.11.2014 KR 20140154488
(62) Divisional of application: 15857492.1
(71) Applicant: Panagene Inc., Daejeon 34027 (KR)
(72) Inventor: CHOI, Bo Ra, 35200 Daejeon (KR); KANG, Jae Hyun, 34322 Daejeon (KR); CHOI, Jae Jin, 34020 Daejeon (KR); KIM, Sung Kee, 34034 Daejeon (KR); PARK, Doo San, 14303 Gwangmyeong-si Gyeonggi-do (KR)
(74) Representative: Maiwald Patent- und Rechtsanwaltsgesellschaft mbH

(57) **Abstract**

The present invention relates to methods for detecting HPV using PNA detection probes. More specifically, it relates to detection probes capable of genotype specifically binding to Human Papillomavirus (hereinafter referred to as "HPV") DNA, detection kits comprising them, methods for detecting HPV genotypes using them. The present invention is capable of accurately detecting 22 HPV genotypes found in cervix, diagnosing coinfection with one or more HPV genotypes, detecting the presence or absence of other HPV genotypes than the 22 genotypes, and detecting HPV genotypes with high sensitivity and specificity.

SEQUENCE LISTING

<110>  PANAGENE INC.

<120>  PNA PROBE, KIT AND METHOD FOR DETECTING HUMAN PAPILLOMAVIRUS

<130>  P15167WOEP

<140>  PCT/KR2015/011478
<141>  2015-10-29

<150>  KR 10-2014-0154488
<151>  2014-11-07

<160>  185

<170>  PatentIn version 3.5

<210>  1
<211>  14
<212>  DNA
<213>  Artificial Sequence

<220>
<223>  HPV genotype detection PNA probe

<400>  1
gcatccgtaa ctac                                                          14


<210>  2
<211>  14
<212>  DNA
<213>  Artificial Sequence

<220>
<223>  HPV genotype detection PNA probe



<220>
<221>  misc_feature
<222>  (4)..(4)
<223>  n= thymine with D-glutamic acid backbone

<220>
<221>  misc_feature
<222>  (8)..(8)
<223>  n= thymine with D-glutamic acid backbone

<220>
<221>  misc_feature
<222>  (12)..(12)
<223>  n= thymine with D-glutamic acid backbone

<400>  2
gcanccgnaa cnac                                                          14


<210>  3
<211>  13
<212>  DNA
<213>  Artificial Sequence

<220>
<223>  HPV genotype detection PNA probe

<400>  3
catccgtaac tac                                                          13


<210>  4
<211>  14
<212>  DNA
<213>  Artificial Sequence

<220>
<223>  HPV genotype detection PNA probe

<400>  4
ctgtgtctaa atct                                                         14


<210>  5
<211>  16
<212>  DNA
<213>  Artificial Sequence

<220>
<223>  HPV genotype detection PNA probe


<220>
<221>  misc_feature
<222>  (4)..(4)
<223>  n= thymine with D-glutamic acid backbone

<220>
<221>  misc_feature
<222>  (8)..(8)
<223>  n= adenine with L-glutamic acid backbone

<400>  5
ctgngtcnaa atctgc                                                       16


<210>  6
<211>  16
<212>  DNA
<213>  Artificial Sequence

<220>
<223>  HPV genotype detection PNA probe

<400>  6
ctgtgtctaa atctgc                                                       16


<210>  7
<211>  15
<212>  DNA
<213>  Artificial Sequence

<220>
<223>  HPV genotype detection PNA probe

<400>  7

ccacatctac ttcag                                                        15


<210>  8
<211>  15
<212>  DNA
<213>  Artificial Sequence

<220>
<223>  HPV genotype detection PNA probe


<220>
<221>  misc_feature
<222>  (6)..(6)
<223>  n= thymine with D-glutamic acid backbone

<220>
<221>  misc_feature
<222>  (8)..(8)
<223>  n= thymine with D-glutamic acid backbone

<220>
<221>  misc_feature
<222>  (12)..(12)
<223>  n= thymine with D-glutamic acid backbone

<400>  8
ccacancnac tncag                                                        15


<210>  9
<211>  17
<212>  DNA
<213>  Artificial Sequence

<220>
<223>  HPV genotype detection PNA probe


<220>
<221>  misc_feature
<222>  (6)..(6)
<223>  n= cytosine with D-glutamic acid backbone

<220>
<221>  misc_feature
<222>  (11)..(11)
<223>  n= cytosine with D-glutamic acid backbone

<400>  9
gctgcnatat ntacttc                                                      17


<210>  10
<211>  18
<212>  DNA
<213>  Artificial Sequence

<220>
<223>  HPV genotype detection PNA probe

<400>  10

acacagtctc ctgtacct                                                    18

<210>  11
<211>  18
<212>  DNA
<213>  Artificial Sequence

<220>
<223>  HPV genotype detection PNA probe

<400>  11
gtctcctgta cctgggca                                                    18

<210>  12
<211>  14
<212>  DNA
<213>  Artificial Sequence

<220>
<223>  HPV genotype detection PNA probe

<400>  12
agtctcctgt acct                                                        14

<210>  13
<211>  14
<212>  DNA
<213>  Artificial Sequence

<220>
<223>  HPV genotype detection PNA probe

<400>  13
gcatctgcat ccac                                                        14

<210>  14
<211>  18
<212>  DNA
<213>  Artificial Sequence

<220>
<223>  HPV genotype detection PNA probe

<400>  14
agcatctgca tccactcc                                                    18

<210>  15
<211>  16
<212>  DNA
<213>  Artificial Sequence

<220>
<223>  HPV genotype detection PNA probe

<400>  15
ttagtacatt atctag                                                      16

<210>  16
<211>  14
<212>  DNA
<213>  Artificial Sequence

<220>
<223>  HPV genotype detection PNA probe

<400>  16
gcacacaagt aact                                                          14


<210>  17
<211>  14
<212>  DNA
<213>  Artificial Sequence

<220>
<223>  HPV genotype detection PNA probe


<220>
<221>  misc_feature
<222>  (10)..(10)
<223>  n= thymine with D-glutamic acid backbone

<220>
<221>  misc_feature
<222>  (14)..(14)
<223>  n= thymine with D-glutamic acid backbone

<400>  17
gcacacaagn aacn                                                          14


<210>  18
<211>  18
<212>  DNA
<213>  Artificial Sequence

<220>
<223>  HPV genotype detection PNA probe

<400>  18
caaaatcctg tgccaagt                                                      18


<210>  19
<211>  15
<212>  DNA
<213>  Artificial Sequence

<220>
<223>  HPV genotype detection PNA probe


<220>
<221>  misc_feature
<222>  (7)..(7)
<223>  n= thymine with D-glutamic acid backbone

<220>
<221>  misc_feature

<222>  (10)..(10)
<223>  n= thymine with D-glutamic acid backbone

<400>  19
acaaaanccn gtgcc                                                        15


<210>  20
<211>  15
<212>  DNA
<213>  Artificial Sequence

<220>
<223>  HPV genotype detection PNA probe


<220>
<221>  misc_feature
<222>  (7)..(7)
<223>  n= thymine with D-glutamic acid backbone

<220>
<221>  misc_feature
<222>  (10)..(10)
<223>  n= thymine with D-glutamic acid backbone

<400>  20
acaaaanccn gtgcc                                                        15


<210>  21
<211>  14
<212>  DNA
<213>  Artificial Sequence

<220>
<223>  HPV genotype detection PNA probe

<400>  21
ggaatacctt cgtc                                                         14


<210>  22
<211>  15
<212>  DNA
<213>  Artificial Sequence

<220>
<223>  HPV genotype detection PNA probe


<220>
<221>  misc_feature
<222>  (5)..(5)
<223>  n= thymine with L-glutamic acid backbone

<220>
<221>  misc_feature
<222>  (8)..(8)
<223>  n= thymine with D-glutamic acid backbone

<400>  22
acctncgnca aggcg                                                        15

<210>  23
<211>  15
<212>  DNA
<213>  Artificial Sequence

<220>
<223>  HPV genotype detection PNA probe


<220>
<221>  misc_feature
<222>  (5)..(5)
<223>  n= thymine with L-glutamic acid backbone

<220>
<221>  misc_feature
<222>  (8)..(8)
<223>  n= thymine with L-glutamic acid backbone

<400>  23
acctncgnca aggcg                                                        15


<210>  24
<211>  17
<212>  DNA
<213>  Artificial Sequence

<220>
<223>  HPV genotype detection PNA probe

<400>  24
gcactgaagt aactaag                                                      17


<210>  25
<211>  14
<212>  DNA
<213>  Artificial Sequence

<220>
<223>  HPV genotype detection PNA probe

<400>  25
ctgaagtaac taag                                                         14


<210>  26
<211>  17
<212>  DNA
<213>  Artificial Sequence

<220>
<223>  HPV genotype detection PNA probe

<400>  26
gcactgaagt aactaag                                                      17


<210>  27
<211>  17

<212>  DNA
<213>  Artificial Sequence

<220>
<223>  HPV genotype detection PNA probe

<400>  27
acaatctgca tctgcca                                                    17


<210>  28
<211>  17
<212>  DNA
<213>  Artificial Sequence

<220>
<223>  HPV genotype detection PNA probe

<400>  28
caatctgcat ctgccac                                                    17


<210>  29
<211>  17
<212>  DNA
<213>  Artificial Sequence

<220>
<223>  HPV genotype detection PNA probe

<400>  29
caatctgcat ctgccac                                                    17


<210>  30
<211>  14
<212>  DNA
<213>  Artificial Sequence

<220>
<223>  HPV genotype detection PNA probe

<400>  30
caggctagta gctc                                                       14


<210>  31
<211>  13
<212>  DNA
<213>  Artificial Sequence

<220>
<223>  HPV genotype detection PNA probe

<400>  31
ctgtatgtgt agg                                                        13


<210>  32
<211>  14
<212>  DNA
<213>  Artificial Sequence

<220>
<223>  HPV genotype detection PNA probe

<400>  32
caggctagta gctc                                                         14


<210>  33
<211>  18
<212>  DNA
<213>  Artificial Sequence

<220>
<223>  HPV genotype detection PNA probe

<400>  33
gcaaacagtg atactaca                                                     18


<210>  34
<211>  17
<212>  DNA
<213>  Artificial Sequence

<220>
<223>  HPV genotype detection PNA probe

<400>  34
gcaaacagtg atactac                                                      17


<210>  35
<211>  14
<212>  DNA
<213>  Artificial Sequence

<220>
<223>  HPV genotype detection PNA probe


<220>
<221>  misc_feature
<222>  (6)..(6)
<223>  n= thymine with L-glutamic acid backbone

<220>
<221>  misc_feature
<222>  (9)..(9)
<223>  n= thymine with L-glutamic acid backbone

<220>
<221>  misc_feature
<222>  (12)..(12)
<223>  n= thymine with L-glutamic acid backbone

<400>  35
aacagngana cnac                                                         14


<210>  36
<211>  19
<212>  DNA
<213>  Artificial Sequence

<220>
<223>  HPV genotype detection PNA probe

<400>  36
tgctgtgtct tctagtgac                                                    19


<210>  37
<211>  16
<212>  DNA
<213>  Artificial Sequence

<220>
<223>  HPV genotype detection PNA probe

<400>  37
tgctgtgtct tctagt                                                       16


<210>  38
<211>  19
<212>  DNA
<213>  Artificial Sequence

<220>
<223>  HPV genotype detection PNA probe

<400>  38
tgctgtgtct tctagtgac                                                    19


<210>  39
<211>  16
<212>  DNA
<213>  Artificial Sequence

<220>
<223>  HPV genotype detection PNA probe

<400>  39
tatagagtct tccata                                                       16


<210>  40
<211>  14
<212>  DNA
<213>  Artificial Sequence

<220>
<223>  HPV genotype detection PNA probe


<220>
<221>  misc_feature
<222>  (6)..(6)
<223>  n= thymine with L-glutamic acid backbone

<220>
<221>  misc_feature
<222>  (9)..(9)
<223>  n= thymine with L-glutamic acid backbone

<220>
<221>  misc_feature
<222>  (13)..(13)
<223>  n= thymine with L-glutamic acid backbone

<400>  40
tagagnctnc cana                                                          14


<210>  41
<211>  16
<212>  DNA
<213>  Artificial Sequence

<220>
<223>  HPV genotype detection PNA probe

<400>  41
tcttccatac cttcta                                                        16


<210>  42
<211>  15
<212>  DNA
<213>  Artificial Sequence

<220>
<223>  HPV genotype detection PNA probe

<400>  42
tgctgcggtt tcccc                                                         15


<210>  43
<211>  15
<212>  DNA
<213>  Artificial Sequence

<220>
<223>  HPV genotype detection PNA probe


<220>
<221>  misc_feature
<222>  (6)..(6)
<223>  n= adenine with L-glutamic acid backbone

<220>
<221>  misc_feature
<222>  (11)..(11)
<223>  n= adenine with L-glutamic acid backbone

<400>  43
actgcngcag nttcc                                                         15


<210>  44
<211>  14
<212>  DNA
<213>  Artificial Sequence

<220>
<223>  HPV genotype detection PNA probe

<400>  44
tttactccaa gtaa                                                          14


<210>  45
<211>  16
<212>  DNA
<213>  Artificial Sequence

<220>
<223>  HPV genotype detection PNA probe

<400>  45
tacatataat tcaaag                                                        16


<210>  46
<211>  17
<212>  DNA
<213>  Artificial Sequence

<220>
<223>  HPV genotype detection PNA probe

<400>  46
ctacatataa ttcaaag                                                       17


<210>  47
<211>  15
<212>  DNA
<213>  Artificial Sequence

<220>
<223>  HPV genotype detection PNA probe

<400>  47
acatataatt caaag                                                         15


<210>  48
<211>  14
<212>  DNA
<213>  Artificial Sequence

<220>
<223>  HPV genotype detection PNA probe

<400>  48
agtattgcta caga                                                          14


<210>  49
<211>  16
<212>  DNA
<213>  Artificial Sequence

<220>
<223>  HPV genotype detection PNA probe

<400>  49
agtactgcta cagaac                                                        16

<210>  50
<211>  15
<212>  DNA
<213>  Artificial Sequence

<220>
<223>  HPV genotype detection PNA probe

<400>  50
ctacagaaca gttaa                                                        15


<210>  51
<211>  16
<212>  DNA
<213>  Artificial Sequence

<220>
<223>  HPV genotype detection PNA probe

<400>  51
gcttctacta cttctt                                                       16


<210>  52
<211>  16
<212>  DNA
<213>  Artificial Sequence

<220>
<223>  HPV genotype detection PNA probe


<220>
<221>  misc_feature
<222>  (4)..(4)
<223>  n= thymine with L-glutamic acid backbone

<220>
<221>  misc_feature
<222>  (9)..(9)
<223>  n= thymine with L-glutamic acid backbone

<400>  52
gctnctacna cttctt                                                       16


<210>  53
<211>  16
<212>  DNA
<213>  Artificial Sequence

<220>
<223>  HPV genotype detection PNA probe


<220>
<221>  misc_feature
<222>  (4)..(4)
<223>  n= thymine with L-glutamic acid backbone

<220>
<221>  misc_feature
<222>  (9)..(9)
<223>  n= thymine with L-glutamic acid backbone

<400>  53
gctnctacna cttctt                                                       16


<210>  54
<211>  16
<212>  DNA
<213>  Artificial Sequence

<220>
<223>  HPV genotype detection PNA probe

<400>  54
caacatgact attaat                                                       16


<210>  55
<211>  16
<212>  DNA
<213>  Artificial Sequence

<220>
<223>  HPV genotype detection PNA probe


<220>
<221>  misc_feature
<222>  (6)..(6)
<223>  n= thymine with L-glutamic acid backbone

<220>
<221>  misc_feature
<222>  (10)..(10)
<223>  n= thymine with L-glutamic acid backbone

<220>
<221>  misc_feature
<222>  (13)..(13)
<223>  n= thymine with L-glutamic acid backbone

<400>  55
caacangacn atnaat                                                       16


<210>  56
<211>  14
<212>  DNA
<213>  Artificial Sequence

<220>
<223>  HPV genotype detection PNA probe

<400>  56
attaatgcag ctaa                                                         14


<210>  57
<211>  15

<212>  DNA
<213>  Artificial Sequence

<220>
<223>  HPV genotype detection PNA probe

<400>  57
tctactactg aatca                                                        15


<210>  58
<211>  14
<212>  DNA
<213>  Artificial Sequence

<220>
<223>  HPV genotype detection PNA probe


<220>
<221>  misc_feature
<222>  (3)..(3)
<223>  n= thymine with L-glutamic acid backbone

<220>
<221>  misc_feature
<222>  (6)..(6)
<223>  n= thymine with L-glutamic acid backbone

<400>  58
tcnacnacta caga                                                         14


<210>  59
<211>  14
<212>  DNA
<213>  Artificial Sequence

<220>
<223>  HPV genotype detection PNA probe


<220>
<221>  misc_feature
<222>  (3)..(3)
<223>  n= thymine with L-glutamic acid backbone

<220>
<221>  misc_feature
<222>  (6)..(6)
<223>  n= thymine with L-glutamic acid backbone

<400>  59
tcnacnacta caga                                                         14


<210>  60
<211>  15
<212>  DNA
<213>  Artificial Sequence

<220>
<223>  HPV genotype detection PNA probe

<400>  60
ctgctgtata tagcc                                                        15


<210>  61
<211>  16
<212>  DNA
<213>  Artificial Sequence

<220>
<223>  HPV genotype detection PNA probe

<400>  61
atagccctac aaagtt                                                       16


<210>  62
<211>  15
<212>  DNA
<213>  Artificial Sequence

<220>
<223>  HPV genotype detection PNA probe

<400>  62
ctgctgtatt tagcc                                                        15


<210>  63
<211>  14
<212>  DNA
<213>  Artificial Sequence

<220>
<223>  HPV genotype detection PNA probe

<400>  63
taaccattag cact                                                         14


<210>  64
<211>  16
<212>  DNA
<213>  Artificial Sequence

<220>
<223>  HPV genotype detection PNA probe

<400>  64
cactgctgtt actcca                                                       16


<210>  65
<211>  15
<212>  DNA
<213>  Artificial Sequence

<220>
<223>  HPV genotype detection PNA probe

<400>  65
gcactgctgt tactc                                                        15

<210>  66
<211>  20
<212>  DNA
<213>  Artificial Sequence

<220>
<223>  DNA oligomer sequences for analysis of characteristics of PNA
       probes - MY11

<220>
<221>  misc_feature
<222>  (3)..(3)
<223>  n is M

<220>
<221>  misc_feature
<222>  (9)..(9)
<223>  n is W

<220>
<221>  misc_feature
<222>  (15)..(15)
<223>  n is Y

<400>  66
gcncagggnc ataanaatgg                                                    20

<210>  67
<211>  20
<212>  DNA
<213>  Artificial Sequence

<220>
<223>  DNA oligomer sequences for analysis of characteristics of PNA
       probes - MY12

<220>
<221>  misc_feature
<222>  (9)..(9)
<223>  n is H

<220>
<221>  misc_feature
<222>  (12)..(12)
<223>  n is Y

<220>
<221>  misc_feature
<222>  (15)..(15)
<223>  n is Y

<400>  67
ggacagggnc anaanaatgg                                                    20

<210>  68
<211>  27
<212>  DNA

<213>  Artificial Sequence

<220>
<223>  DNA oligomer sequences for analysis of characteristics of PNA
       probes

<400>  68
tgcgtcatgt ggaagagtat gatttac                                          27


<210>  69
<211>  26
<212>  DNA
<213>  Artificial Sequence

<220>
<223>  DNA oligomer sequences for analysis of characteristics of PNA
       probes

<400>  69
tgtaaatcat actcttccac atgacg                                           26


<210>  70
<211>  23
<212>  DNA
<213>  Artificial Sequence

<220>
<223>  DNA oligomer sequences for analysis of characteristics of PNA
       probes

<400>  70
gaatacatgc gccatgtgga gga                                              23


<210>  71
<211>  24
<212>  DNA
<213>  Artificial Sequence

<220>
<223>  DNA oligomer sequences for analysis of characteristics of PNA
       probes

<400>  71
gcgcatgtat tccttataat ctga                                             24


<210>  72
<211>  26
<212>  DNA
<213>  Artificial Sequence

<220>
<223>  DNA oligomer sequences for analysis of characteristics of PNA
       probes


<220>
<221>  misc_feature
<222>  (12)..(12)
<223>  n is R

<400>  72
actaacttta angagtacct acgaca                                          26


<210>  73
<211>  22
<212>  DNA
<213>  Artificial Sequence

<220>
<223>  DNA oligomer sequences for analysis of characteristics of PNA
       probes


<220>
<221>  misc_feature
<222>  (2)..(2)
<223>  n is Y

<400>  73
cntccccatg tcgtaggtac tc                                              22


<210>  74
<211>  26
<212>  DNA
<213>  Artificial Sequence

<220>
<223>  DNA oligomer sequences for analysis of characteristics of PNA
       probes

<400>  74
ctaccaaatt taagcagtat agcaga                                          26


<210>  75
<211>  30
<212>  DNA
<213>  Artificial Sequence

<220>
<223>  DNA oligomer sequences for analysis of characteristics of PNA
       probes

<400>  75
tctgctatac tgcttaaatt tggtagcatc                                      30


<210>  76
<211>  28
<212>  DNA
<213>  Artificial Sequence

<220>
<223>  DNA oligomer sequences for analysis of characteristics of PNA
       probes

<400>  76
gacatggcga agaatatgaa ttacaatt                                        28

<210>  77
<211>  29
<212>  DNA
<213>  Artificial Sequence

<220>
<223>  DNA oligomer sequences for analysis of characteristics of PNA
       probes

<400>  77
tctgttgtaa gtgttatttt acacaactg                                         29


<210>  78
<211>  28
<212>  DNA
<213>  Artificial Sequence

<220>
<223>  DNA oligomer sequences for analysis of characteristics of PNA
       probes


<220>
<221>  misc_feature
<222>  (19)..(19)
<223>  n is Y

<400>  78
catgttgaag aatatgatnt acagtttg                                          28


<210>  79
<211>  30
<212>  DNA
<213>  Artificial Sequence

<220>
<223>  DNA oligomer sequences for analysis of characteristics of PNA
       probes


<220>
<221>  misc_feature
<222>  (10)..(10)
<223>  n is R

<400>  79
caaactgtan atcatattct tcaacatgtc                                        30


<210>  80
<211>  29
<212>  DNA
<213>  Artificial Sequence

<220>
<223>  DNA oligomer sequences for analysis of characteristics of PNA
       probes

<400>  80
agtagacatg tggaggaata tgatttaca                                         29

<210>  81
<211>  22
<212>  DNA
<213>  Artificial Sequence

<220>
<223>  DNA oligomer sequences for analysis of characteristics of PNA
       probes

<400>  81
ctccacatgt ctactatact gc                                                22


<210>  82
<211>  27
<212>  DNA
<213>  Artificial Sequence

<220>
<223>  DNA oligomer sequences for analysis of characteristics of PNA
       probes



<220>
<221>  misc_feature
<222>  (26)..(26)
<223>  n is R

<400>  82
tacaatttat ttttcaattg tgcaana                                           27


<210>  83
<211>  28
<212>  DNA
<213>  Artificial Sequence

<220>
<223>  DNA oligomer sequences for analysis of characteristics of PNA
       probes



<220>
<221>  misc_feature
<222>  (4)..(4)
<223>  n is Y

<220>
<221>  misc_feature
<222>  (16)..(16)
<223>  n is Y

<400>  83
agcngttaat gtaatnttgc acaattga                                          28


<210>  84
<211>  29
<212>  DNA
<213>  Artificial Sequence

<220>

<223>  DNA oligomer sequences for analysis of characteristics of PNA
       probes

<220>
<221>  misc_feature
<222>  (11)..(11)
<223>  n is R

<400>  84
atgttgaaga ntatgactta cagtttgtt                                          29

<210>  85
<211>  29
<212>  DNA
<213>  Artificial Sequence

<220>
<223>  DNA oligomer sequences for analysis of characteristics of PNA
       probes

<220>
<221>  misc_feature
<222>  (19)..(19)
<223>  n is Y

<400>  85
aacaaactgt aagtcatant cttcaacat                                          29

<210>  86
<211>  21
<212>  DNA
<213>  Artificial Sequence

<220>
<223>  DNA oligomer sequences for analysis of characteristics of PNA
       probes

<400>  86
cagtttataa ggcatggtga g                                                  21

<210>  87
<211>  23
<212>  DNA
<213>  Artificial Sequence

<220>
<223>  DNA oligomer sequences for analysis of characteristics of PNA
       probes

<400>  87
tcctcaccat gccttataaa ctg                                                23

<210>  88
<211>  27
<212>  DNA
<213>  Artificial Sequence

<220>
<223>  DNA oligomer sequences for analysis of characteristics of PNA
      probes

<400>  88
agacatgcag aagagtttga tttacag                                          27


<210>  89
<211>  36
<212>  DNA
<213>  Artificial Sequence

<220>
<223>  DNA oligomer sequences for analysis of characteristics of PNA
      probes

<400>  89
tagtagaatt catagaatgt atatatgtca ttacct                                36


<210>  90
<211>  28
<212>  DNA
<213>  Artificial Sequence

<220>
<223>  DNA oligomer sequences for analysis of characteristics of PNA
      probes


<220>
<221>  misc_feature
<222>  (17)..(17)
<223>  n is Y

<400>  90
gacatggtga ggaattngat ttacaatt                                         28


<210>  91
<211>  26
<212>  DNA
<213>  Artificial Sequence

<220>
<223>  DNA oligomer sequences for analysis of characteristics of PNA
      probes


<220>
<221>  misc_feature
<222>  (2)..(2)
<223>  n is R

<220>
<221>  misc_feature
<222>  (19)..(19)
<223>  n is K

<400>  91
cnaattcctc accatgtcnt aaatac                                           26

<210>  92
<211>  29
<212>  DNA
<213>  Artificial Sequence

<220>
<223>  DNA oligomer sequences for analysis of characteristics of PNA
       probes

<400>  92
cttctaagtt taaggaatat accaggcac                                          29


<210>  93
<211>  22
<212>  DNA
<213>  Artificial Sequence

<220>
<223>  DNA oligomer sequences for analysis of characteristics of PNA
       probes

<400>  93
cttcaccatg ccttaaatat tc                                                 22


<210>  94
<211>  30
<212>  DNA
<213>  Artificial Sequence

<220>
<223>  DNA oligomer sequences for analysis of characteristics of PNA
       probes


<220>
<221>  misc_feature
<222>  (21)..(21)
<223>  n is Y

<400>  94
gacatgttga agaatatgat ntacagtttg                                         30


<210>  95
<211>  28
<212>  DNA
<213>  Artificial Sequence

<220>
<223>  DNA oligomer sequences for analysis of characteristics of PNA
       probes

<400>  95
cgtgcctggt atattcctta aacttaga                                           28


<210>  96
<211>  28
<212>  DNA
<213>  Artificial Sequence

<220>
<223>  DNA oligomer sequences for analysis of characteristics of PNA
      probes

<400>  96
gaatatttaa ggcatggtga agaatatg                                          28


<210>  97
<211>  28
<212>  DNA
<213>  Artificial Sequence

<220>
<223>  DNA oligomer sequences for analysis of characteristics of PNA
      probes

<400>  97
ctaatatatt gcttaaagtt acttggag                                          28


<210>  98
<211>  28
<212>  DNA
<213>  Artificial Sequence

<220>
<223>  DNA oligomer sequences for analysis of characteristics of PNA
      probes

<400>  98
catgcagagg aatatgaatt acaatttg                                          28


<210>  99
<211>  25
<212>  DNA
<213>  Artificial Sequence

<220>
<223>  DNA oligomer sequences for analysis of characteristics of PNA
      probes


<220>
<221>  misc_feature
<222>  (14)..(14)
<223>  n is Y

<400>  99
attcctctgc atgnctaaca tactg                                             25


<210>  100
<211>  30
<212>  DNA
<213>  Artificial Sequence

<220>
<223>  DNA oligomer sequences for analysis of characteristics of PNA
      probes

<220>
<221>  misc_feature
<222>  (26)..(26)
<223>  n is R

<400>  100
atcagtacct tagacatgtg gaggantatg                                        30


<210>  101
<211>  23
<212>  DNA
<213>  Artificial Sequence

<220>
<223>  DNA oligomer sequences for analysis of characteristics of PNA
       probes

<400>  101
ctccacatgt ctaaggtact gat                                               23


<210>  102
<211>  26
<212>  DNA
<213>  Artificial Sequence

<220>
<223>  DNA oligomer sequences for analysis of characteristics of PNA
       probes

<400>  102
cagttttaaa gaatatgcca gacatg                                            26


<210>  103
<211>  23
<212>  DNA
<213>  Artificial Sequence

<220>
<223>  DNA oligomer sequences for analysis of characteristics of PNA
       probes

<400>  103
gtctggcata ttctttaaaa ctg                                               23


<210>  104
<211>  28
<212>  DNA
<213>  Artificial Sequence

<220>
<223>  DNA oligomer sequences for analysis of characteristics of PNA
       probes

<400>  104
tgaaatcaat caataccttc gccatgtg                                          28


<210>  105

<211>  25
<212>  DNA
<213>  Artificial Sequence

<220>
<223>  DNA oligomer sequences for analysis of characteristics of PNA
       probes

<400>  105
catggcgaag gtattgattg atttc                                          25


<210>  106
<211>  27
<212>  DNA
<213>  Artificial Sequence

<220>
<223>  DNA oligomer sequences for analysis of characteristics of PNA
       probes

<400>  106
ttaggcatgt tgaggaatat gatttgc                                        27


<210>  107
<211>  27
<212>  DNA
<213>  Artificial Sequence

<220>
<223>  DNA oligomer sequences for analysis of characteristics of PNA
       probes

<400>  107
caaatcatat tcctcaacat gcctaac                                        27


<210>  108
<211>  25
<212>  DNA
<213>  Artificial Sequence

<220>
<223>  DNA oligomer sequences for analysis of characteristics of PNA
       probes

<400>  108
gcatgtggag gaatatgatt tacaa                                          25


<210>  109
<211>  22
<212>  DNA
<213>  Artificial Sequence

<220>
<223>  DNA oligomer sequences for analysis of characteristics of PNA
       probes

<400>  109
tccacatgcc tagtatattc ct                                             22

<210>  110
<211>  28
<212>  DNA
<213>  Artificial Sequence

<220>
<223>  DNA oligomer sequences for analysis of characteristics of PNA
       probes

<400>  110
caatttatat ttcaattgtg taaaatca                                          28


<210>  111
<211>  23
<212>  DNA
<213>  Artificial Sequence

<220>
<223>  DNA oligomer sequences for analysis of characteristics of PNA
       probes

<400>  111
tagaatccat ggtgtgcagg taa                                               23


<210>  112
<211>  20
<212>  DNA
<213>  Artificial Sequence

<220>
<223>  Primer and probe system for detection of HPV genotypes - GP6

<400>  112
tgatttacag tttatttttc                                                   20


<210>  113
<211>  20
<212>  DNA
<213>  Artificial Sequence

<220>
<223>  Primer and probe system for detection of HPV genotypes - MY09


<220>
<221>  misc_feature
<222>  (6)..(6)
<223>  n is M

<220>
<221>  misc_feature
<222>  (8)..(8)
<223>  n is R

<220>
<221>  misc_feature
<222>  (9)..(9)
<223>  n is R

<220>
<221>  misc_feature
<222>  (13)..(13)
<223>  n is W

<400>  113
cgtccnanng ganactgatc                                                   20


<210>  114
<211>  20
<212>  DNA
<213>  Artificial Sequence

<220>
<223>  Primer and probe system for detection of HPV genotypes -
       detection PNA

<400>  114
tttgttactg tggtagatac                                                   20


<210>  115
<211>  20
<212>  DNA
<213>  Artificial Sequence

<220>
<223>  Primer and probe system for detection of HPV genotypes -
       detection PNA

<400>  115
tttgttacag ttgtggatac                                                   20


<210>  116
<211>  20
<212>  DNA
<213>  Artificial Sequence

<220>
<223>  Primer and probe system for detection of HPV genotypes -
       detection PNA

<400>  116
tttgtaactg ttgtagatac                                                   20


<210>  117
<211>  20
<212>  DNA
<213>  Artificial Sequence

<220>
<223>  Primer and probe system for detection of HPV genotypes -
       detection PNA

<400>  117
tttattacct gtgttgatac                                                   20


<210>  118
<211>  20

<212>  DNA
<213>  Artificial Sequence

<220>
<223>  Primer and probe system for detection of HPV genotypes -
       detection PNA

<400>  118
tttgtaactg ttgtggatac                                                    20


<210>  119
<211>  20
<212>  DNA
<213>  Artificial Sequence

<220>
<223>  Primer and probe system for detection of HPV genotypes -
       detection PNA

<400>  119
tttttaactg tggtggacac                                                    20


<210>  120
<211>  20
<212>  DNA
<213>  Artificial Sequence

<220>
<223>  Primer and probe system for detection of HPV genotypes -
       detection PNA

<400>  120
tttattactt gtgttgacac                                                    20


<210>  121
<211>  20
<212>  DNA
<213>  Artificial Sequence

<220>
<223>  Primer and probe system for detection of HPV genotypes -
       detection PNA

<400>  121
tttgtcacgg tggtagatac                                                    20


<210>  122
<211>  17
<212>  DNA
<213>  Artificial Sequence

<220>
<223>  Primer and probe system for detection of HPV genotypes - specific
       primer

<400>  122
cagggccaca ataatgg                                                       17

<210>  123
<211>  20
<212>  DNA
<213>  Artificial Sequence

<220>
<223>  Primer and probe system for detection of HPV genotypes - specific
       primer

<400>  123
gcgcagggcc acaataatgg                                                   20


<210>  124
<211>  15
<212>  DNA
<213>  Artificial Sequence

<220>
<223>  Primer and probe system for detection of HPV genotypes - specific
       primer

<400>  124
gcgcaaggcc acaat                                                        15


<210>  125
<211>  20
<212>  DNA
<213>  Artificial Sequence

<220>
<223>  Primer and probe system for detection of HPV genotypes - specific
       primer

<400>  125
gcgcaaggcc acaataatgg                                                   20


<210>  126
<211>  17
<212>  DNA
<213>  Artificial Sequence

<220>
<223>  Primer and probe system for detection of HPV genotypes - specific
       primer

<400>  126
cccagggcca caataat                                                      17


<210>  127
<211>  20
<212>  DNA
<213>  Artificial Sequence

<220>
<223>  Primer and probe system for detection of HPV genotypes - specific
       primer

<400>  127
gcccagggcc acaataatgg                                                   20

<210>  128
<211>  19
<212>  DNA
<213>  Artificial Sequence

<220>
<223>  Primer and probe system for detection of HPV genotypes - specific
       primer

<400>  128
catttgcttc tgacacaac                                                    19


<210>  129
<211>  24
<212>  DNA
<213>  Artificial Sequence

<220>
<223>  Primer and probe system for detection of HPV genotypes - specific
       primer

<400>  129
gcttacattt gcttctgaca caac                                              24


<210>  130
<211>  18
<212>  DNA
<213>  Artificial Sequence

<220>
<223>  Primer and probe system for detection of HPV genotypes - specific
       primer

<400>  130
ctgtaattca tattcctc                                                     18


<210>  131
<211>  26
<212>  DNA
<213>  Artificial Sequence

<220>
<223>  Primer and probe system for detection of HPV genotypes - specific
       primer

<400>  131
ctgtaattca tattcctcta catgtc                                            26


<210>  132
<211>  20
<212>  DNA
<213>  Artificial Sequence

<220>
<223>  Primer and probe system for detection of HPV genotypes - specific
       primer

<400>  132
gcacatcata ttcttcagca                                                   20


<210>  133
<211>  24
<212>  DNA
<213>  Artificial Sequence

<220>
<223>  Primer and probe system for detection of HPV genotypes - specific
       primer

<400>  133
gcacatcata ttcttcagca tgtc                                              24


<210>  134
<211>  20
<212>  DNA
<213>  Artificial Sequence

<220>
<223>  Primer and probe system for detection of HPV genotypes - specific
       primer

<400>  134
aaatcaaact cctctgtatg                                                   20


<210>  135
<211>  26
<212>  DNA
<213>  Artificial Sequence

<220>
<223>  Primer and probe system for detection of HPV genotypes - specific
       primer

<400>  135
tggcaaatca aactcctctg tatggc                                            26


<210>  136
<211>  16
<212>  DNA
<213>  Artificial Sequence

<220>
<223>  Primer and probe system for detection of HPV genotypes - specific
       primer

<400>  136
aaatcaaatt cctccg                                                       16


<210>  137
<211>  24
<212>  DNA
<213>  Artificial Sequence

<220>
<223>  Primer and probe system for detection of HPV genotypes - specific

primer

<400>  137
gcaaatcaaa ttcctccgtg tgtc                                              24


<210>  138
<211>  17
<212>  DNA
<213>  Artificial Sequence

<220>
<223>  Primer and probe system for detection of HPV genotypes - specific
       primer

<400>  138
tcatattcct ctgtgtg                                                      17


<210>  139
<211>  26
<212>  DNA
<213>  Artificial Sequence

<220>
<223>  Primer and probe system for detection of HPV genotypes - specific
       primer

<400>  139
gtaagtcata ttcctctgtg tggcgg                                            26


<210>  140
<211>  19
<212>  DNA
<213>  Artificial Sequence

<220>
<223>  Primer and probe system for detection of HPV genotypes - specific
       primer

<400>  140
catccacgtt caccttgcc                                                    19


<210>  141
<211>  21
<212>  DNA
<213>  Artificial Sequence

<220>
<223>  Primer and probe system for detection of HPV genotypes - specific
       primer

<400>  141
ttcatccacg ttcaccttgc c                                                 21


<210>  142
<211>  24
<212>  DNA
<213>  Artificial Sequence

<220>
<223>  Primer and probe system for detection of HPV genotypes - specific
      primer



<220>
<221>  misc_feature
<222>  (2)..(2)
<223>  n is Y

<220>
<221>  misc_feature
<222>  (5)..(5)
<223>  n is Y

<220>
<221>  misc_feature
<222>  (7)..(7)
<223>  n is I

<220>
<221>  misc_feature
<222>  (8)..(8)
<223>  n is K

<220>
<221>  misc_feature
<222>  (12)..(12)
<223>  n is W

<220>
<221>  misc_feature
<222>  (14)..(14)
<223>  n is Y

<220>
<221>  misc_feature
<222>  (17)..(17)
<223>  n is Y

<220>
<221>  misc_feature
<222>  (20)..(20)
<223>  n is I

<220>
<221>  misc_feature
<222>  (21)..(21)
<223>  n is I

<400>  142
antgnanntc anantcntcn ncat                                              24


<210>  143
<211>  27
<212>  DNA
<213>  Artificial Sequence

<220>
<223>  Primer and probe system for detection of HPV genotypes - specific
      primer

<220>
<221>  misc_feature
<222>  (4)..(4)
<223>  n is Y

<220>
<221>  misc_feature
<222>  (7)..(7)
<223>  n is Y

<220>
<221>  misc_feature
<222>  (9)..(9)
<223>  n is I

<220>
<221>  misc_feature
<222>  (10)..(10)
<223>  n is K

<220>
<221>  misc_feature
<222>  (14)..(14)
<223>  n is W

<220>
<221>  misc_feature
<222>  (16)..(16)
<223>  n is Y

<220>
<221>  misc_feature
<222>  (19)..(19)
<223>  n is Y

<220>
<221>  misc_feature
<222>  (22)..(22)
<223>  n is I

<220>
<221>  misc_feature
<222>  (23)..(23)
<223>  n is I

<400>  143
aaantgnann tcanantcnt cnncatg                                          27


<210>  144
<211>  22
<212>  DNA
<213>  Artificial Sequence

<220>
<223>  Primer and probe system for detection of HPV genotypes - specific
      primer


<220>
<221>  misc_feature
<222>  (2)..(2)

<223>  n is Y

<220>
<221>  misc_feature
<222>  (5)..(5)
<223>  n is Y

<220>
<221>  misc_feature
<222>  (8)..(8)
<223>  n is D

<220>
<221>  misc_feature
<222>  (12)..(12)
<223>  n is W

<220>
<221>  misc_feature
<222>  (14)..(14)
<223>  n is Y

<220>
<221>  misc_feature
<222>  (17)..(17)
<223>  n is Y

<220>
<221>  misc_feature
<222>  (20)..(20)
<223>  n is I

<220>
<221>  misc_feature
<222>  (21)..(21)
<223>  n is I

<400>  144
antgnaantc anantcntcn nc                                                22


<210>  145
<211>  27
<212>  DNA
<213>  Artificial Sequence

<220>
<223>  Primer and probe system for detection of HPV genotypes - specific
      primer


<220>
<221>  misc_feature
<222>  (4)..(4)
<223>  n is Y

<220>
<221>  misc_feature
<222>  (7)..(7)
<223>  n is Y

<220>
<221>  misc_feature

<222>  (10)..(10)
<223>  n is D

<220>
<221>  misc_feature
<222>  (14)..(14)
<223>  n is W

<220>
<221>  misc_feature
<222>  (16)..(16)
<223>  n is Y

<220>
<221>  misc_feature
<222>  (19)..(19)
<223>  n is Y

<220>
<221>  misc_feature
<222>  (22)..(22)
<223>  n is I

<220>
<221>  misc_feature
<222>  (23)..(23)
<223>  n is I

<400>  145
aaantgnaan tcanantcnt cnncatg                                          27


<210>  146
<211>  23
<212>  DNA
<213>  Artificial Sequence

<220>
<223>  Primer and probe system for detection of HPV genotypes - specific
       primer


<220>
<221>  misc_feature
<222>  (3)..(3)
<223>  n is Y

<220>
<221>  misc_feature
<222>  (10)..(10)
<223>  n is W

<220>
<221>  misc_feature
<222>  (12)..(12)
<223>  n is Y

<220>
<221>  misc_feature
<222>  (15)..(15)
<223>  n is Y

<220>

<221>  misc_feature
<222>  (18)..(18)
<223>  n is I

<220>
<221>  misc_feature
<222>  (19)..(19)
<223>  n is I

<400>  146
tgnaaatcan antcntcnnc atg                                              23


<210>  147
<211>  27
<212>  DNA
<213>  Artificial Sequence

<220>
<223>  Primer and probe system for detection of HPV genotypes - specific
       primer


<220>
<221>  misc_feature
<222>  (4)..(4)
<223>  n is Y

<220>
<221>  misc_feature
<222>  (7)..(7)
<223>  n is Y

<220>
<221>  misc_feature
<222>  (14)..(14)
<223>  n is W

<220>
<221>  misc_feature
<222>  (16)..(16)
<223>  n is Y

<220>
<221>  misc_feature
<222>  (19)..(19)
<223>  n is Y

<220>
<221>  misc_feature
<222>  (22)..(22)
<223>  n is I

<220>
<221>  misc_feature
<222>  (23)..(23)
<223>  n is I

<400>  147
aaantgnaaa tcanantcnt cnncatg                                          27


<210>  148

<211>  19
<212>  DNA
<213>  Artificial Sequence

<220>
<223>  Primer and probe system for detection of HPV genotypes -
       fluorescent probe PNA

<400>  148
ccacacaaac gttatccac                                                    19


<210>  149
<211>  17
<212>  DNA
<213>  Artificial Sequence

<220>
<223>  Primer and probe system for detection of HPV genotypes -
       fluorescent probe PNA

<400>  149
ccacacaaac gttatcc                                                      17


<210>  150
<211>  19
<212>  DNA
<213>  Artificial Sequence

<220>
<223>  Primer and probe system for detection of HPV genotypes -
       fluorescent probe PNA

<400>  150
tactgtaaca actgaagac                                                    19


<210>  151
<211>  18
<212>  DNA
<213>  Artificial Sequence

<220>
<223>  Primer and probe system for detection of HPV genotypes -
       fluorescent probe PNA

<400>  151
ctactgtaac aactgaag                                                     18


<210>  152
<211>  20
<212>  DNA
<213>  Artificial Sequence

<220>
<223>  Primer and probe system for detection of HPV genotypes -
       fluorescent probe PNA

<400>  152
caatccacaa gtacaactgc                                                   20

<210>  153
<211>  17
<212>  DNA
<213>  Artificial Sequence

<220>
<223>  Primer and probe system for detection of HPV genotypes -
       fluorescent probe PNA

<400>  153
gtaggtacac aatccac                                                      17


<210>  154
<211>  14
<212>  DNA
<213>  Artificial Sequence

<220>
<223>  Primer and probe system for detection of HPV genotypes -
       fluorescent probe PNA

<400>  154
acaccaaccc cata                                                         14


<210>  155
<211>  18
<212>  DNA
<213>  Artificial Sequence

<220>
<223>  Primer and probe system for detection of HPV genotypes -
       fluorescent probe PNA

<400>  155
ccacaccaac cccatata                                                     18


<210>  156
<211>  17
<212>  DNA
<213>  Artificial Sequence

<220>
<223>  Primer and probe system for detection of HPV genotypes -
       fluorescent probe PNA

<400>  156
tgcaacatct ggtgata                                                      17


<210>  157
<211>  19
<212>  DNA
<213>  Artificial Sequence

<220>
<223>  Primer and probe system for detection of HPV genotypes -
       fluorescent probe PNA

<400>  157

ctgcaacatc tggtgatac                                                    19


<210>  158
<211>  19
<212>  DNA
<213>  Artificial Sequence

<220>
<223>  Primer and probe system for detection of HPV genotypes -
       fluorescent probe PNA

<400>  158
ctctactgac cctactgtg                                                    19


<210>  159
<211>  17
<212>  DNA
<213>  Artificial Sequence

<220>
<223>  Primer and probe system for detection of HPV genotypes -
       fluorescent probe PNA

<400>  159
ctctactgac cctactg                                                      17


<210>  160
<211>  15
<212>  DNA
<213>  Artificial Sequence

<220>
<223>  Primer and probe system for detection of HPV genotypes -
       fluorescent probe PNA

<400>  160
cacagtcccc tccgt                                                        15


<210>  161
<211>  17
<212>  DNA
<213>  Artificial Sequence

<220>
<223>  Primer and probe system for detection of HPV genotypes -
       fluorescent probe PNA

<400>  161
tacacagtcc cctccgt                                                      17


<210>  162
<211>  15
<212>  DNA
<213>  Artificial Sequence

<220>
<223>  Primer and probe system for detection of HPV genotypes -
       fluorescent probe PNA

<400>  162
tccacgcagg atagc                                                        15


<210>  163
<211>  20
<212>  DNA
<213>  Artificial Sequence

<220>
<223>  Primer and probe system for detection of HPV genotypes -
       fluorescent probe PNA

<400>  163
cagcatccac gcaggatagc                                                   20


<210>  164
<211>  16
<212>  DNA
<213>  Artificial Sequence

<220>
<223>  Primer and probe system for detection of HPV genotypes -
       fluorescent probe PNA

<400>  164
caactcagtc tccatc                                                       16


<210>  165
<211>  14
<212>  DNA
<213>  Artificial Sequence

<220>
<223>  Primer and probe system for detection of HPV genotypes -
       fluorescent probe PNA

<400>  165
caactcagtc tcca                                                         14


<210>  166
<211>  16
<212>  DNA
<213>  Artificial Sequence

<220>
<223>  Primer and probe system for detection of HPV genotypes -
       fluorescent probe PNA

<400>  166
   cccccctgta tctgaa                                                      16


<210>  167
<211>  14
<212>  DNA
<213>  Artificial Sequence

<220>

<223>  Primer and probe system for detection of HPV genotypes -
       fluorescent probe PNA

<400>  167
cctgtatctg aata                                                         14


<210>  168
<211>  12
<212>  DNA
<213>  Artificial Sequence

<220>
<223>  Primer and probe system for detection of HPV genotypes -
       fluorescent probe PNA

<400>  168
ctgcagcaga at                                                           12


<210>  169
<211>  18
<212>  DNA
<213>  Artificial Sequence

<220>
<223>  Primer and probe system for detection of HPV genotypes -
       fluorescent probe PNA

<400>  169
cgcctccact gctgcagc                                                     18


<210>  170
<211>  12
<212>  DNA
<213>  Artificial Sequence

<220>
<223>  Primer and probe system for detection of HPV genotypes -
       fluorescent probe PNA

<400>  170
atcagaggct ac                                                           12


<210>  171
<211>  14
<212>  DNA
<213>  Artificial Sequence

<220>
<223>  Primer and probe system for detection of HPV genotypes -
       fluorescent probe PNA

<400>  171
tcagaggcta cata                                                         14


<210>  172
<211>  18
<212>  DNA
<213>  Artificial Sequence

<220>
<223>  Primer and probe system for detection of HPV genotypes -
       fluorescent probe PNA

<400>  172
ctcacaatcg ccttctgc                                                    18


<210>  173
<211>  15
<212>  DNA
<213>  Artificial Sequence

<220>
<223>  Primer and probe system for detection of HPV genotypes -
       fluorescent probe PNA

<400>  173
cacaatcgcc ttctg                                                       15


<210>  174
<211>  16
<212>  DNA
<213>  Artificial Sequence

<220>
<223>  Primer and probe system for detection of HPV genotypes -
       fluorescent probe PNA

<400>  174
cagctacatc tgctgc                                                      16


<210>  175
<211>  14
<212>  DNA
<213>  Artificial Sequence

<220>
<223>  Primer and probe system for detection of HPV genotypes -
       fluorescent probe PNA

<400>  175
cagctacatc tgct                                                        14


<210>  176
<211>  15
<212>  DNA
<213>  Artificial Sequence

<220>
<223>  Primer and probe system for detection of HPV genotypes -
       fluorescent probe PNA

<400>  176
cagctgctgc tacac                                                       15


<210>  177
<211>  13

<212>  DNA
<213>  Artificial Sequence

<220>
<223>  Primer and probe system for detection of HPV genotypes -
       fluorescent probe PNA

<400>  177
cagctgctgc tac                                                          13


<210>  178
<211>  15
<212>  DNA
<213>  Artificial Sequence

<220>
<223>  Primer and probe system for detection of HPV genotypes -
       fluorescent probe PNA

<400>  178
ccaccaccga atcag                                                        15


<210>  179
<211>  16
<212>  DNA
<213>  Artificial Sequence

<220>
<223>  Primer and probe system for detection of HPV genotypes -
       fluorescent probe PNA

<400>  179
caccaccgaa tcagaa                                                       16


<210>  180
<211>  17
<212>  DNA
<213>  Artificial Sequence

<220>
<223>  Primer and probe system for detection of HPV genotypes -
       fluorescent probe PNA

<400>  180
cactcaaaca accactg                                                      17


<210>  181
<211>  16
<212>  DNA
<213>  Artificial Sequence

<220>
<223>  Primer and probe system for detection of HPV genotypes -
       fluorescent probe PNA

<400>  181
ctcaaacaac cactga                                                       16

<210>  182
<211>  14
<212>  DNA
<213>  Artificial Sequence

<220>
<223>  Primer and probe system for detection of HPV genotypes -
       fluorescent probe PNA

<400>  182
accctctgac acat                                                         14


<210>  183
<211>  12
<212>  DNA
<213>  Artificial Sequence

<220>
<223>  Primer and probe system for detection of HPV genotypes -
       fluorescent probe PNA

<400>  183
cctctgacac at                                                           12


<210>  184
<211>  17
<212>  DNA
<213>  Artificial Sequence

<220>
<223>  Primer and probe system for detection of HPV genotypes -
       fluorescent probe PNA

<400>  184
agtctgccgt tactgcc                                                      17


<210>  185
<211>  15
<212>  DNA
<213>  Artificial Sequence

<220>
<223>  Primer and probe system for detection of HPV genotypes -
       fluorescent probe PNA

<400>  185
gtctgccgtt actgc                                                        15

## Description

### TECHNICAL FIELD

The present invention relates to methods for detecting genotypes of Human Papillomavirus (hereinafter referred to as "HPV") using PNA detection probes containing a fluorescent substance and a quencher. More specifically, it relates to methods for real-time simultaneous detection of multiple Human Papillomavirus HPV genotypes using one or more detection probes capable of genotype-specifically binding to HPV DNA, analysis of a melting curve, and HPV genotype analysis kits involving analysis of a melting curve.

### BACKGROUND ART

Human Papillomavirus (hereinafter referred to as "HPV") has double stranded circular DNA of 8 kb, which codes for eight genes of E1, E2, E4, E5, E6, E7, L1 and L2. HPV infects epithelial cells, and is implicated with warts and various malignant tumors, in mammals including human beings. Actually, HPV infection has been reported to be the most common cause of cervical cancer (see Howley PM. Virology, 1996, 2:2045-2109).

Cervical cancer is a malignant tumor of cervix, and constitutes 95% of all uterine cancers. Breast cancer has been reported the most frequent cancer, and cervical cancer the second most frequent cancer, among female cancers all over the world (Korea Centers for Disease Control and Prevention, 2004). Usually, cervical cancer begins with HPV infection in basal epithelial cells of cervix, and then, progresses to infiltrating cancer after long preneoplastic phase including low squamous intraepithelial lesion (LSIL), high squamous intraepithelial lesion (HSIL) and intraepithelial neoplasia. Since cervical cancer develops stepwise over a long period of time, if preneoplastic lesion in the intermediate phase of cervical cancer is effectively diagnosed, it can be treated prior to progression to infiltrating cancer, thereby enabling the prevention of cervical cancer. Therefore, it is very important for diagnosis, prevention and treatment of cervical cancer to provide a method for effectively detecting HPV, which is the cause of cervical cancer.

HPV genotype is determined depending on the open reading frame of E6, E7 and L1 genes, among the genomic sequence of HPV. Heretofore, more than 120 different genotypes have been identified. Those genotypes can be classified into high-risk group and low-risk group, depending on the risk of causing cervical cancer. HPV 16, 18, 31, 33, 35, 39, 45, 51, 52, 56, 58, 59 and 68, which are in the high-risk group, HPV 6, 11, 26, 34, 40, 42, 44, 47, 53, 66 and 69, which are in the low-risk group, and HPV 26, 73 and 82, which are in the potential high-risk group, are implicated in the development of cervical cancer (see Comparison of Seegene Anyplex II HPV 28 with the PGMY-CHUV Assay for Human papillomavirus Genotyping , C. Estrade et al., J Clin Microbiol, 2014, 52(2): 607). As shown above, genotypes of HPV infected significantly affect the progress of cancer. Therefore, detection of presence of HPV in a sample and of its genotype is important for prognosis and diagnosis of cervical cancer.

Methods for detection of presence of HPV and of its genotype are generally classified into papanicolaou smear (or pap smear), direct identification of HPV DNA, and ones involving amplification of HPV DNA. Pap smear is for the primary screening of cervical cancer and of preneoplastic lesion on the basis of the cytomorphology of pap smear. The method has been performed as a standard test from 1940, and used up to now for diagnosis of HPV. However, the accuracy of the test relies upon the skill of the tester, and is reported to have high false negative rate of 30-40% (see Ledger WJ et al., Am J Obstet Gynecol, 2000, 182: 860-865).

Direct identification of HPV DNA includes liquid hybridization include liquid hybridization (Hybrid Capture by Digene Diagnostics, Silver Spring, MD), southern blot and dot blot using HPV type-specific probes, and filter in situ hybridization (FISH) (see Detection of high-risk HPV type by the hybrid Capture 2 test, George et al., J Clin Microbiol, 2001, 65:155-162). However, the methods has a disadvantage of low test sensitivity because they do not involve amplification of DNA of HPV.

The methods using amplification of HPV DNA include type-specific polymerase chain reaction and general primer PCR. In particular, screening of genotypes is generally performed by dot blot hybridization, microtiter plate hybridization, line probe assay (see Evaluation of a Modified Reverse Line Blot Assay for Detection and Typing of Human Papillomavirus, Feray et al., 2005, Am J clin Pathol. 123:896-899) or the like from HPV DNA amplified with general primer set (see Leen-Jan et al., J Clin Microbiol, 2006, 3292-3298]. However, those methods involve complicated procedures, are time consuming and labor intensive because a number of samples must be treated and analyzed, and have low detection sensitivity and difficulties in data interpretation [see Gravitt PE et al., J Clin Microbiol, 1998, 36:3020-3027].

Recently, detection kits of HPV genotypes have been developed based on DNA microarray (DNA chip) technology (see Korean Patent Laid-Open Nos. 2006-0015669 and 2004-0078506). The kits have DNA probes specific to various genotypes of HPV immobilized onto a glass slide, and can simultaneously detect their hybridization reactions with PCR-amplified products. Since the HPV DNA chip enables rapid detection of various genotypes of HPV within a short time, it has been commercially utilized widely for the diagnosis of HPV.

Further, a method for HPV diagnosis using bead microarray has been recently developed. The method includes the immobilization of HPV genotype-specific probes on beads and their hybridization with target nucleic acids in one tube. Since HPV genotypes are detected by selecting beads having probes hybridized with target nucleic acids, a number of samples can be treated within a shorter time than conventional DNA chips which have probes immobilized on a glass slide or the like. In addition, the method is very commercially useful, because it enables detection of HPV genotypes with increased specificity and decreased cost (see Facile, comprehensive, High-Throughput Genotyping of Human Genital Papillomaviruses using spectrally addressable Liquid Bead Microarrays, Jan Wallace et al., 2005, J. Mol. Diagn, 7: 72-80 and Bead-based multiplex genotyping of Human Papillomaviruses, Markus et al., 2006, J. Clin. Microbiol, 44: 504-512). Optical methods using fluorophores that emit fluorescence of specific wavelength are generally used to confirm whether DNA/DNA hybridization occurs between DNA of specific genotype and immobilized DNA probe. Further, other known methods such as electrochemical methods may also be used. However, the DNA chip or bead array has low stability because of low biological or chemical stability of immobilized DNA probes themselves to nucleases, etc., and so may have denatured DNA and decreased reactivity upon long-term storage (see Korean Patent Laid-Open No. 2006-0091708).

In order to overcome the instability of DNA itself, various DNA analogues have been developed. Among them, PNAs (peptide nucleic acids) have been developed by Nielsen in 1991. In PNA, phosphodiester bonds of DNA are replaced by peptide bonds. Since a PNA has adenine, thymine, guanine and cytosine like DNA, it can perform base-specific hybridization with DNA or RNA. In particular, its difference in structure of the backbone, which is modified with peptide bonds, alters anionic property of phosphate backbone of DNA or RNA to neutral. The removal of electrostatic repulsion between anions resulting from neutralization of anionic property directly contributes to the increase in binding ability upon hybridization. As a result, it has increased hybridization rate and specificity, and thus, has improved S/N (signal to noise) ratio. In addition, PNA is more stable than DNA or RNA because biological degrading enzymes such as nucleases cannot recognize PNA (see Peptide nucleic acid, PNA, sequence-selective recognition of DNA by strand displacement with a thymine-substituted polyamide, P.E. Nielsen et al., 1991, Science, 254, 1497-1500).

As described above, PNA, which has high hybridization ability and stability while retaining the functions of DNA or RNA, is recognized as a promising alternative to DNA that can complement drawbacks of DNA. Thus, extensive studies have been conducted for analysis or diagnosis with PNA oligomers in place of DNA oligomers (Korean Patent Nos. 91708 and 12544 and reference: [Peptide nucleic acids on microarrays and other biosensors, Brandt O et al., 2004, Trends in Biotechnology, 22, 617-622], [Detection of target DNA using fluorescent cationic polymer and peptide nucleic acid probes on solid support, Fredric R Raymond et al., 2005, BMC technology, 5, 1-5]).

For example, the inventors of the present invention filed a patent application, Korean Patent Application No. 10-2007-0022201, which relates to PNA chips and methods for detecting HPV genotypes using probes having excellent hybridization binding ability and stability. However, even though the PNA chips has an advantage of accurate detection of HPV genotypes with high sensitivity and specificity, due to its user convenience and long duration of reaction, there has been in increasing need for detection of HPV genotypes by real-time PCR.

In order to solve the problem, a test method is necessary that is capable of analyzing multiple samples accurately in a short period of time with small amount of labor. It can be said that, among the techniques currently available, the one that satisfies the conditions the most is real-time PCR. Real-time PCR, which monitors increase in the PCR-amplified products in real time in every cycle, is an analysis technique for detection of a fluorescent substance reacting with the PCR-amplified products and quantitatively. When compared to conventional PCR where staining is performed on gel after the final step and the PCR-amplified products are observed after electrophoresis, this method does not involve an additional operation of electrophoresis and has excellent accuracy and sensitivity and high reproducibility. In addition, it is a diagnostic method that can be automated and quantifiable. It has an excellent biological stability against contamination from staining agents such as EtBr (Ethidium Bromide) and ultraviolet radiation and allows automatic identification of the presence or absence of amplification of specific genes and accurate quantification of DNA and RNA to be detected (see Real-time PCR in virology, Mackay I.M., Arden K.E. and Nitsche A., 2002, Nucleic Acids Res. 30(6): 1292-1305).

However, in real-time PCT as above, only genotypes of 16 and 18 can be detected due to difference in design of a fluorescent substance, and only up to four genotypes can be identified in one tube because genotypes are detected based on fluorescence only. Efforts have continuously been made to detect various gene variations in a tube, and analysis of a melting curve using DNA probes has been used. The DNA probes used for analysis of a melting curve include MGB Taqman probe, molecular beacon (MB), and binary probe. These methods have an advantage that they are capable of identifying a single nucleotide variation but has a drawback that the minimal length of a DNA probe is at 20-40mer, which is long, making it difficult to detect single nucleotide variations that are adjacent to one another in one reaction (see Hidefumi Sasaki et al., 2005, Clin Cancer Res. 11: 2924-2929, Manna Zhang et al., 2002, HEPATOLOGY 36: 3).

### DETAILED DESCRIPTION OF THE INVENTION

### TECHNICAL SUBJECT MATTER

In order to solve the above-mentioned problems of prior arts, the inventors of the present application have prepared PNA probes that are capable of specifically binding to each of the genotypes of HPV using PNA having the above-mentioned advantages. The HPV genotype detection PNA probes include the ones that can detect 20 genotypes in the high-risk group (types 16, 18, 26, 31, 33, 35, 39, 45, 51, 52, 53, 56, 58, 59, 66, 68, 69, 70, 73 and 82 in the high-risk group), two genotypes in the low-risk group (types 6 and 11 in the low-risk group), and other 18 genotypes that are frequently found in Koreans (types 30, 32, 34, 40, 42, 43, 44, 54, 55, 61, 62, 67, 74, 81, 83, 84, 87 and 90). In addition, HPV-positive probes that can be hybridized with the consensus region of each HPV genotype have been prepared to confirm HPV infection.

Using the PNA probes, real-time polymerase chain reaction kits have been produced. In addition, using HPV consensus primer combination that is capable of raising amplification sensitivity of HPV, the inventors have confirmed that each genotype could be detected with high specificity and sensitivity, and thus, completed the present invention.

Given the above, the object of the present invention is provision of PNA probes that are capable of accurately detecting HPV genotypes with high specificity and sensitivity and are stable against biological degrading enzymes. Another object of the present invention is provision of detection kits for detecting HPV genotypes comprising the probes. The other object of the present invention is provision of methods for detecting HPV genotypes using the detection kits.

### MEANS FOR ACHIEVING THE SUBJECT MATTER

PNA, which is excellent, in comparison with DNA, in thermal and biological stability and in recognition and binding ability for target DNA, can be used as probes for real-time PCR for detection of a single nucleotide variation. However, real-time PCR uses a fluorescent substance for detection of target DNA in a sample, and thus, in order to detect various genotypes, probes that have two different wavelengths are necessary. This issue is a major obstacle for multiplex detection. In the present invention, the issue can be solved by analysis of a melting curve of fluorescent probes.

The present invention provides PNA probes to which a reporter and a quencher for detecting each of 22 HPV genotypes are linked. In addition, it provides PNA probes to which a reporter and a quencher for detecting other HPV genotypes than the 22 HPV genotypes are linked.

The present invention provides a melting curve analysis method using PNA probes to which a reporter and a quencher for detecting HPV genotypes are linked.

The present invention provides kits for detecting HPV genotypes comprising the HPV PNA probes.

The PNA probes used for analysis of a melting curve for detecting HPV genotypes may be general probes or have a negatively charged molecule. When PNA probes having a negative charge are used, resolution of a melting curve increases, making it easy to analyze various genotypes in a reaction tube at once.

Additionally, the PNA probes may include a fluorescent substance. The fluorescent substance may be a reporter, a quencher and an intercalating fluorescent substance.

### EFFECT OF THE INVENTION

By using the method for detecting HPV, it is possible to simultaneously analyze real-time amplification curves and melting curves for 20 HPV genotypes in the high-risk group and two HPV genotypes in the low-risk group, which allows accurate multiplex identification of HPV genotypes. In addition, it is possible to detect HPV genotypes other than the 22 types that can be identified and to confirm the presence of absence of infection with HPV. In addition, since each genotype can be detected with high specificity and sensitivity, the method can be quite useful for identification of HPV genotypes.

### SIMPLE DESCRIPTION OF THE DRAWINGS

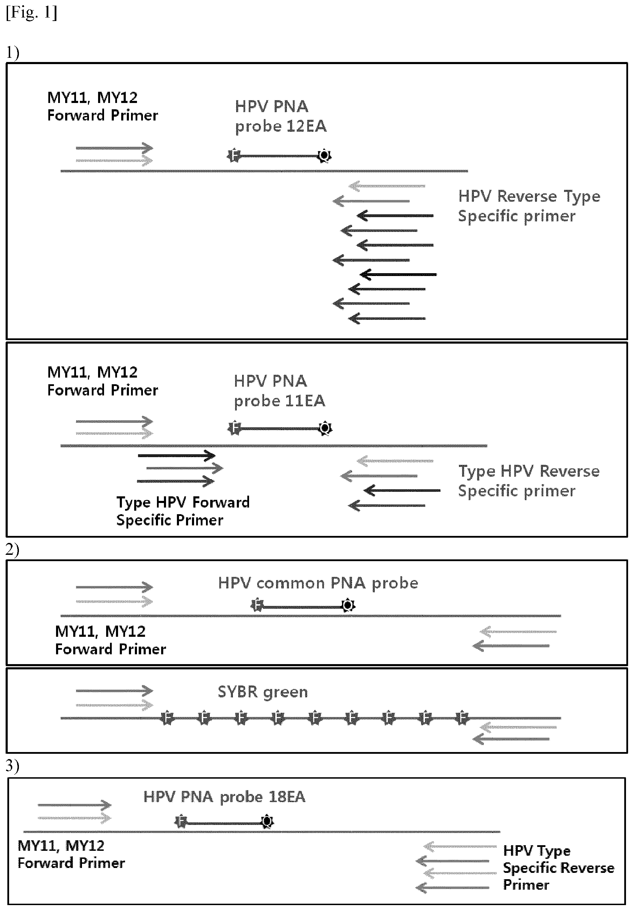

Item 1) of Fig. 1 is a schematic diagram showing a multiplex detection probe and an HPV type-specific primer mixture. Item 2) of Fig. 1 is a schematic diagram showing a primer mixture using a SYBR green or fluorescent PNA detection probe for identification of HPV infection and other types. Item 3) of Fig. 1 a schematic diagram showing a primer complex using a fluorescent PNA probe for identification of the 18 HPV genotypes (30, 32, 34, 40, 42, 43, 44, 54, 55, 61, 62, 67, 74, 81, 83, 84, 87 and 90) other than 22 HPV genotypes (6, 11, 16, 18, 26, 31, 33, 35, 39, 45, 51, 52, 53, 56, 58, 59, 66, 68, 69, 70, 73 and 82).

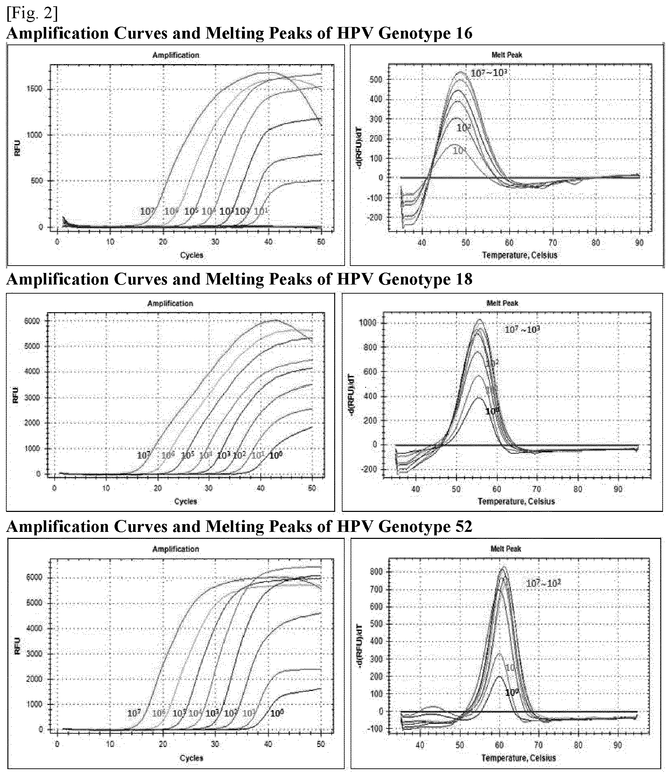

Fig. 2 is data showing detection sensitivity and melting peaks of HPV genotypes by using mixtures of fluorescent PNA detection probes and primers. Detection sensitivity of specificity of HPV genotypes have been identified by using HPV fluorescent PNA probe mixtures to be detected and HPV type-specific primer mixtures. By analysis of the melting peak of each fluorescent probe, it was possible to detect a substance having one copy in HPV DNA, and it has been confirmed, by using the Tm value of the fluorescent substances labeled on the detection probes, that each genotype is specifically identified.

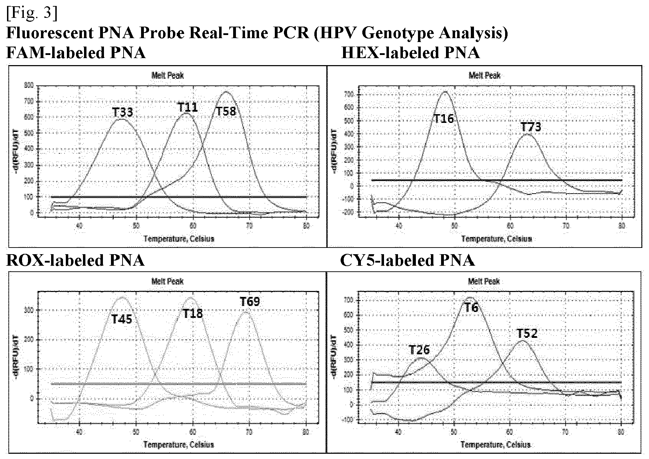

In Fig. 3, which involves mixtures of fluorescent PNA detection probes and primers, it has been confirmed that HPV genotypes are identified fluorescent substances and the Tm value. It has been confirmed that, by labelling each fluorescent substance (FAM, HEX, ROX, CY5) on 11 types of PNAs, that genotypes are identified by Tm. It has been confirmed that respective PNA mixtures labeled with the fluorescent substance FAM can identify genotypes 33, 11 and 58 and have the Tm of 47.0°C, 58.5°C and 66.0° C. In addition, it has been confirmed that respective PNA mixtures labeled with the fluorescent substance HEX can identify genotypes 16 and 73 and have the probe Tm of 48.0°C and 63°C. It has been confirmed that respective PNA mixtures labeled with the fluorescent substance ROX can identify genotypes 45, 18 and 69 and have the Tm of 47.5 ° C, 59.5 ° C and 69.0 ° C. It has been confirmed that respective PNA mixtures labeled with the fluorescent substance CY5 can identify genotypes 26, 6 and 52 and Tm detected at 44.0°C, 53.0°C, 62.5°C.

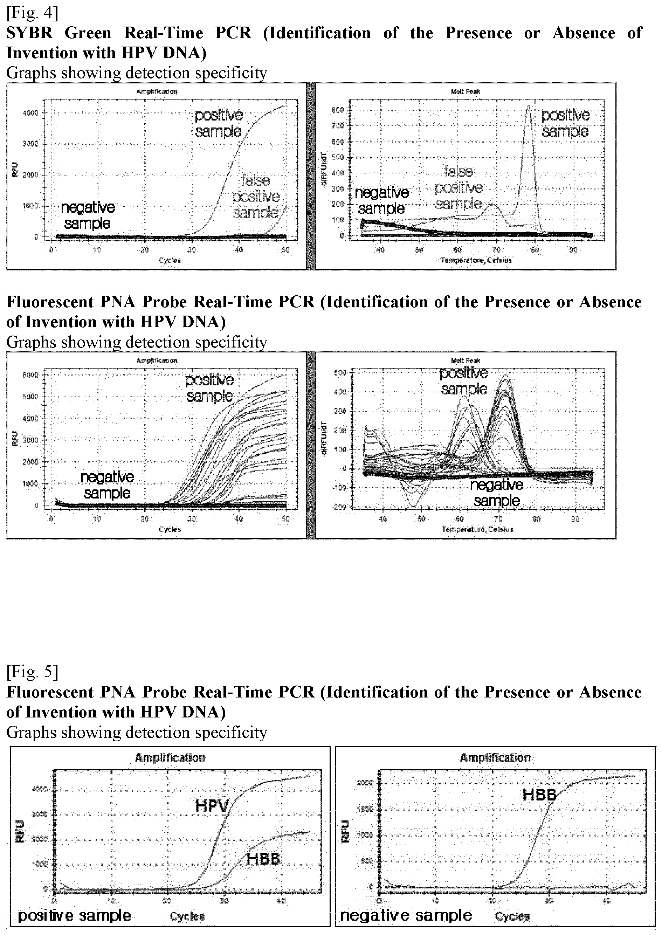

In Fig. 4, screening for HPV infection was conducted by analyzing Ct (cycle threshold) graphs obtained from real-time PCR by using SYBR green to determine whether HPV infection is present or not, and it has been confirmed that it is possible to verify specificity of amplification of HPV genes by analysis of melting peaks. In addition, it has been confirmed that the fluorescent probe detection system aimed to identify the presence or absence of HPV infection is capable of identifying the presence or absence of infection with HPV DNA and verifying specificity through analysis of Ct graphs and melting peaks.

In Fig. 5, it has been confirmed that it is possible to detect HPV genotypes though analysis of the Ct graphs obtained from real-time PCR by using the fluorescent PNA probes for detection of 18 HPV genotypes (types 30, 32, 34, 40, 42, 43, 44, 54, 55, 61, 62, 67, 74, 81, 83, 84, 87 and 90).

### BEST MODE FOR CARRYING OUT THE INVENTION

Unless defined otherwise, all technical and scientific terms used in the present specification have the same meaning as the one that is commonly understood by a skilled expert in the technical field to which the present invention pertains. Normally, the terms used in the present specification are well known in the technical field and are commonly used.

The present invention relates to methods for simultaneously detecting HPV genotypes by using HPV genotype detection probes to which a reporter and a quencher are linked.

The detection probes of the present invention indicate all nucleic acids and nucleic acid analogues that complementarily bind with HPV-specific genotypes and can be selected from the group consisting of oligonucleotide, PNAs (peptide nucleic acids) and LNA (locked nucleic acid). The polymerases for PCR normally exhibit nuclease activity, which may cause damage to probes. It is thus preferable to use synthetic nucleic acids such as PNA that are stable to a nuclease. In addition, since detection probes play the role of selectively detect a desired target nucleic acid gene, it is possible to use probes for detecting a nucleic acid that are known in the pertinent technical field. Even when the detection probes of the present invention are nucleic acid analogues other than PNA, analyses were performed using MGB-taqman detection probes in confirm whether detection of the target nucleic acid is possible. As a result, it can be confirmed by real-time amplification curve that MGB-taqman probes for detecting mutants and a wide-type clamping PNA probe system operate in different strands.

In the present invention, preferably, PNA probes are used. PNA probes, which are synthetic DNA, are capable of specifically binding with the target HPV DNA or RNA and are stable with respect to a nuclease. It is accordingly possible to analyze a melting curve based on the probes.

Additionally, in the present invention, the detection probes can be put under a steric change by linking specific functional groups such as a side chain of a natural amino acid or synthetic amino acid in N-terminus or C- terminus of the probe or alpha, beta, gamma or linker position of the probe backbone. The amino acid may be characterized in that it has no charge or has a negative charge or a positive charge, but it is not limited thereto, and it is possible to use a method for alteration of a steric structure a probe and a charge imparting method known in the field.

The HPV genotype detection PNA probes of the present invention include the ones that can detect 20 genotypes in the high-risk group (types 16, 18, 26, 31, 33, 35, 39, 45, 51, 52, 53, 56, 58, 59, 66, 68, 69, 70, 73 and 82 in the high-risk group), two genotypes in the low-risk group (types 6 and 11 in the low-risk group), and other 18 genotypes that are frequently found in Koreans (types 30, 32, 34, 40, 42, 43, 44, 54, 55, 61, 62, 67, 74, 81, 83, 84, 87 and 90). In an example of the present invention, PNA detection probes can detect a HPV genotype consisting of any one of sequences of SEQ ID NO: 1 to 65, 114 to 121, and 148 to 185 and is selected from the groups consisting of genotypes 6, 11, 16, 18, 26, 30, 31, 32, 33, 34, 35, 39, 40, 42, 43, 44, 45, 51, 52, 53, 54, 55, 56, 58, 59, 61, 62, 66, 67, 68, 69, 70, 73, 74, 81, 82, 83, 84, 87 and 90.

The HPV genotype detection PNA probes of the present invention may be general probes or have a negatively charged molecule. When PNA probes having a negative charge is used, resolution of a melting curve increases, making it easy to analyze various genotypes in a reaction tube at once.

PNA probes according to the present invention including a negatively charged molecule may include at least one negatively charged molecule selected from all groups of phosphoric acid, carboxylic acid, sulphonic acid, nitric acid and boric acid. The negatively charged molecules may be linked to N-terminus or C- terminus of the probe or alpha, beta or gamma position of the PNA backbone. At least one negatively charged molecule may bind to a base of the probe continuously or intermittently.

The PNA probes according to the present invention can be applied to various HPV genotype detection techniques due to their characteristic of a narrow half width at half maximum of melting curve graphs. In addition, due to a high resolution of the melting curves, melting curves are distinguished according to the sequence of each HPV genotype, which allows simple identification of genotype without additional analysis such as sequencing analysis of each HPV genotype.

A reporter and a quencher that can quench fluorescence may bind at both ends of the detection probes of the present invention, and the probes can include an intercalating fluorescent substance.

The 'reporter' is a substance that absorbs and emits light of specific wavelength to emit fluorescence and refers to a substance that is labeled on a probe to confirm hybridization between the target nucleic acid and the probe. It may be at least one selected from the group consisting of fluorescein, fluorescein chlorotriazinyl, rhodamine green, rhodamine red, tetramethylrhodamine, FITC, Oregon green, Alexa Fluor, FAM, JOE, ROX, HEX, Texas Red, TET, TRITC, TAMRA, a cyanine-based dye, and a thiadicarbocyanine dye. Preferably, the reporter may be at least one selected from the group consisting of FAM (6-carboxyfluorescein), Texas red, JOE, TAMRA, CY5 and CY3 .

The 'quencher' refers to a substance that absorbs light generated by the reporter to reduce strength of fluorescence and may be at least one selected from the group consisting of Dabcyl, TAMRA, Eclipse, DDQ, QSY, Blackberry Quencher, Black Hole Quencher, Qxl, Iowa black FQ, Iowa black RQ and IRDye QC-1. It is preferable to use TAMRA (6-carboxytetramethyl-rhodamine), BHQ1, BHQ2 or Dabsyl as the quencher, but it is not limited thereto. Difference types of quenchers have difference degree of reduction in fluorescence; thus, quenchers may be used in this consideration.

In an example, a PNA probe including a negatively charged molecule, a reporter and a quencher emits a fluorescence signal after being hybridized with the target DNA. As temperature goes up, it melts with the target DNA at the temperature suitable for melting of the probe by negative charge effect to quench the fluorescence signal. The PNA probe including a negative charge, when compared with a PNA probe without a negative charge, is dissociated quickly from the target DNA to increase resolution of a melting curve. By analysis of the high-resolution melting curve obtained from the fluorescence signal due to the temperature change, the HPV genotype of the target DNA can be identified.

The 'intercalating fluorescent substance' may be selected from the group consisting of acridine homodimer and its derivatives, Acridine Orange and its derivatives, 7-aminoactinomycin D (7-AAD) and its derivatives, Actinomycin D and its derivatives, ACMA (9-amino-6-chloro-2-methoxyacridine) and its derivatives, DAPI and its derivatives, Dihydroethidium and its derivatives, Ethidium bromide and its derivatives, EthD-1 and its derivatives, EthD-2 and its derivatives, Hexidium iodide and its derivatives, bisbenzimide (Hoechst 33258) and its derivatives, Hoechst 33342 and its derivatives, Hoechst 34580 and its derivatives, hydroxystilbamidine and its derivatives, LDS 751 and its derivatives, Propidium Iodide (PI) and its derivatives, and Cy-dyes derivatives.

In an example, a probe linked with a negatively charged molecule and an intercalating fluorescent substance emits a fluorescence signal after being hybridized with the target DNA. It quickly melts with the target DNA at the temperature suitable for melting of the probe by negative charge effect to quench the signal. By analysis of the high-resolution melting curve of the fluorescence signal due to the temperature, HPV genotypes can be identified.

Additionally, the target HPV genotype analysis according to the present invention may include analysis of a melting curve obtained from fluorescent signal detection without direct link between an intercalating fluorescent substance and a probe.

In general, conventional amplification curve analysis using real-time PCR, in order to simultaneously detect multiple HPV genotypes, uses a fluorescent substance in detecting the target nucleic acid in a sample. Accordingly, there was a problem that multiplex detection was limited because, for detection of two or more target nucleic acids, probes that have fluorescent substances as many as the number of the targets. However, the simultaneous multiplex HPV genotype detection method of the present invention is characterized in that multiple HPV genotypes can be detected using one fluorescent substance because an amplification curve and a melting curve analysis can be simultaneously performed using detection probes.

Additionally, the simultaneous multiplex HPV genotype detection method using the probes of the present invention is not limited to simultaneous analysis of an amplification curve and a melting curve, but an amplification curve and a melting curve can be analyzed separately or sequentially. Only one of an amplification curve and a melting curve may be analyzed as necessary to detect the target nucleic acid. In particular, when quantitative analysis is not required, the presence or absence of the target nucleic acid, or the genotype thereof can be identified by analysis of a melting curve without analysis of an amplification curve.

The simultaneous multiplex HPV genotype detection methods of the present invention specifically comprises (a) performing hybridization by mixing a detection probe and a primer in the sample which contains the target nucleic acid to obtain a real-time amplification curve; (b) obtaining a melting curve between an amplified product and the detection probe with changing temperature after the amplification; and (c) analyzing the obtained real-time amplification curve and melting curve separately, sequentially or simultaneously.

The detection probe of the step (a) may be adjusted variously according to the HPV genotype to be detected. The present invention may be characterized in that the step of obtaining an amplification curve or a melting curve was performed by real-time PCR (polymerase chain reaction) and analysis of the amplification curve was performed by measuring the value of Ct (cycle threshold). When the target nucleic acid is present in a sample or the sample has a large amount of the target nucleic acid, the number of cycles reaching the threshold goes down and the measured value Ct is low, from which the absence or the genotype of the target nucleic acid can be identified and the initial amount of the target nucleic acid can be measured.

In addition, the analysis of a melting curve, in general, is the last process that is performed after completion of real-time PCR. The temperature of a sample is dropped to 30 to 55°C and then the intensity of fluorescence signal is measured as the temperature is increased by 1°C per half a second to 95°C. When the detection probe and the target nucleic acid (a strand of the target nucleic acid that can complementarily bind with the detection probe) are separated as the temperature rises, fluorescence is quenched to rapidly drop the fluorescence signal, which allows identification, from the melting peak, of the presence or absence of the target nucleic acid.

The simultaneous multiplex HPV detection methods of the present invention may be characterized in that detection is possible in a nucleic acid sample having 10¹ or less copies.

Upon analyzing, according to the methods of the present invention, real-time amplification curves and melting curves of HPV genotypes 16, 18 and 52 for at different concentrations from 10⁷ to 10⁰ copies, it has been confirmed that, as shown in Figs. 2 to 5, the HPV genotypes having 10⁰ copies can be detected and that multiple simultaneous detections are possible.

The kits of the present invention may selectively include a buffer, a DNA polymerase cofactor and a reagent, including deoxyribonucleotide-5-triphosphate, that is necessary to perform a target amplifying PCR reaction (e.g., PCR reaction). Alternatively, the kits of the present application may also comprise various polynucleotide molecules, a reverse transcriptase, various buffers and reagents, and an antibody inhibiting DNA polymerase activity.

Additionally, the optimal amount of the reagent of the kit used for a specific reaction can be easily determined by a skilled person understanding the matters stated in the present specification. Typically, the kits of the present invention are prepared as a separate package or compartment including the constituents mentioned above.

### [Examples]

The present invention is hereinafter described in further detail by way of examples. The examples are only examples of the present invention and it is obvious for a person having ordinary skill in the pertinent field that the scope of the present invention is not limited by the examples.

### [Analysis of detection probes and DNA oligomers]

### [Example 1]

### Examples of the PNA probes and target DNA oligomers used for HPV detection

### 1-1: Preparation of PNA probes

The PNA probes used in the present invention were intended to detect various targets by labeling detection probes which have conformational change and structural change by impartment of charges, and general probes. In order to confirm the detection and specificity of various HPV genotypes by using PNA oligomers, PNA probes were synthesized as in Table 1. As fluorescent PNA probes for detection of HPV genotypes, probes with sequences that were specific to HPV genotypes to be detected were used, where D-glutamic acid and L-glutamic acid having a negative charge in gamma position of the backbone of probes that were not modified and the PNA probes.

**[Table 1]**

| Sequence of the probes used in the present invention | | | | |
|---|---|---|---|---|
| SEQ ID NO | PNA name | PNA probe sequence | | |
| | | N-terminus | PNA sequence (N→C) | C-terminus |
| 1 | fluorescent probe HPV 6-1 | Dabcyl | GCATCCGTAACTAC | OEK(HEX) |
| 2 | fluorescent probe HPV 6-2 | Dabcyl | GCA②CCG②AAC②AC | OEK(HEX) |
| 3 | fluorescent probe HPV 6-3 | Dab-K(Dab) | CATCCGTAACTAC | OEK(Cv5) |
| 4 | fluorescent probe HPV 11-1 | Dabcvl | CTGTGTCTAAATCT | OEK(HEX) |
| 5 | fluorescent probe HPV 11-2 | Dabcyl | CTG②GTC1AAATCTGC | OEK(HEX) |
| 6 | fluorescent probe HPV 11-3 | Dabcyl | CTGTGTCTAAATCTGC | OK(FAM) |
| 7 | fluorescent probe HPV 16-1 | Dabcyl | CCACATCTACTTCAG | OEK(HEX) |
| 8 | fluorescent probe HPV 16-2 | Dabcyl | CCACA②C②ACT②CAG | OEK(HEX) |
| 9 | fluorescent probe HPV 16-3 | Dabcyl | GCTGC④ATAT④TACTTC | OEK(HEX) |
| 10 | fluorescent probe HPV 18-1 | Dabcyl | ACACAGTCTCCTGTACCT | OEK(ROX) |
| 11 | fluorescent probe HPV 18-2 | Dabcyl | GTCTCCTGTACCTGGGCA | OEK(ROX) |
| 12 | fluorescent probe HPV 18-3 | Dabcyl | AGTCTCCTGTACCT | OEK(ROX) |
| 13 | fluorescent probe HPV 26-1 | Dabcyl | GCATCTGCATCCAC | OEK(CY5) |
| 14 | fluorescent probe HPV 26-2 | Dabcyl | AGCATCTGCATCCACTCC | OEK(CY5) |
| 15 | fluorescent probe HPV 26-3 | Dab-K(Dab) | TTAGTACATTATCTAG | OEK(Cy5) |
| 16 | fluorescent probe HPV 33-1 | Dabcyl | GCACACAAGTAACT | OEK(F AM) |
| 17 | fluorescent probe HPV 33-2 | Dabcyl | GCACACAAG(2)AAC(2) | OEK(F AM) |
| 18 | fluorescent probe HPV 45-1 | Dabcyl | CAAAATCCTGTGCCAAGT | OEK(ROX) |
| 19 | fluorescent probe HPV 45-2 | Dabcyl | ACAAAA(2)CC(2)GTGCC | OEK(ROX) |
| 20 | fluorescent probe HPV 45-3 | Dabcyl | ACAAAA(2)CC(2)GTGCC | OEK(ROX) |
| 21 | fluorescent probe HPV 52-1 | Dabcyl | GGAATACCTTCGTC | OEK(F AM) |
| 22 | fluorescent probe HPV 52-2 | Dabcyl | ACCT2CG②CAAGGCG | OEK(F AM) |
| 23 | fluorescent probe HPV 52-3 | Dab-K(Dab) | ACCT2CG2CAAGGCG | OEK(Cy5) |
| 24 | fluorescent probe HPV 58-1 | Dabcyl | GCACTGAAGTAACTAAG | OEK(F AM) |
| 25 | fluorescent probe HPV 58-2 | Dabcyl | CTGAAGTAACTAAG | OEK(F AM) |
| 26 | fluorescent probe HPV 58-3 | Dabcyl | GCACTGAAGTAACTAAG | OK(FAM) |
| 27 | fluorescent probe HPV 69-1 | Dabcyl | ACAATCTGCATCTGCCA | OEK(HEX) |
| 28 | fluorescent probe HPV 69-2 | Dabcyl | CAATCTGCATCTGCCAC | OEK(HEX) |
| 29 | fluorescent probe HPV 69-3 | Dabcyl | CAATCTGCATCTGCCAC | OEK(ROX) |
| 30 | fluorescent probe HPV 73-1 | Dabcyl | CAGGCTAGTAGCTC | OEK(F AM) |
| 31 | fluorescent probe HPV 73-2 | Dabcyl | CTGTATGTGTAGG | OEK(F AM) |
| 32 | fluorescent probe HPV 73-3 | Dabcyl | CAGGCTAGTAGCTC | OEK(HEX) |
| 33 | fluorescent probe HPV 31-1 | Dabcyl | GCAAACAGTGATACTACA | OEK(F AM) |
| 34 | fluorescent probe HPV 31-2 | Dabcyl | GCAAACAGTGATACTAC | OEK(F AM) |
| 35 | fluorescent probe HPV 31-3 | Dabcyl | AACAG2GA2AC2AC | OEK(HEX) |
| 36 | fluorescent probe HPV 35-1 | Dabcyl | TGCTGTGTCTTCTAGTGAC | OEK(HEX) |
| 37 | fluorescent probe HPV 35-2 | Dabcyl | TGCTGTGTCTTCTAGT | OEK(HEX) |
| 38 | fluorescent probe HPV 35-3 | Dabcyl | TGCTGTGTCTTCTAGTGAC | OK(FAM) |
| 39 | fluorescent probe HPV 39-1 | Dabcyl | TATAGAGTCTTCCATA | OEK(ROX) |
| 40 | fluorescent probe HPV 39-2 | Dabcyl | TAGAG2CT2CCA2A | OEK(ROX) |
| 41 | fluorescent probe HPV 39-3 | Dabcyl | TCTTCCATACCTTCTA | OEK(HEX) |
| 42 | fluorescent probe HPV 51-1 | Dabcyl | TGCTGCGGTTTCCCC | OEK(F AM) |
| 43 | fluorescent probe HPV 51-2 | Dabcyl | ACTGC1GCAG1TTCC | OEK(F AM) |
| 44 | fluorescent probe HPV 51-3 | Dab-K(Dab) | TTTACTCCAAGTAA | OEK(Cy5) |
| 45 | fluorescent probe HPV 53-1 | Dabcyl | TACATATAATTCAAAG | OEK(HEX) |
| 46 | fluorescent probe HPV 53-2 | Dabcyl | CTACATATAATTCAAAG | OEK(HEX) |
| 47 | fluorescent probe HPV 53-3 | Dabcyl | ACATATAATTCAAAG | OK(FAM) |
| 48 | fluorescent probe HPV 56-1 | Dabcyl | AGTATTGCTACAGA | OEK(HEX) |
| 49 | fluorescent probe HPV 56-2 | Dabcyl | AGTACTGCTACAGAAC | OEK(HEX) |
| 50 | fluorescent probe HPV 56-3 | Dabcyl | CTACAGAACAGTTAA | OK(ROX) |
| 51 | fluorescent probe HPV 59-1 | Dabcyl | GCTTCTACTACTTCTT | OEK(ROX) |
| 52 | fluorescent probe HPV 59-2 | Dabcyl | GCT2CTAC2ACTTCTT | OEK(ROX) |
| 53 | fluorescent probe HPV 59-3 | Dabcyl | GCT2CTAC2ACTTCTT | OEK(HEX) |
| 54 | fluorescent probe HPV 66-1 | Dabcyl | CAACATGACTATTAAT | OEK(F AM) |
| 55 | fluorescent probe HPV 66-2 | Dabcyl | CAACA2GAC2AT2AAT | OEK(F AM) |
| 56 | fluorescent probe HPV 66-3 | Dab-K(Dab) | ATTAATGCAGCTAA | OEK(Cy5) |
| 57 | fluorescent probe HPV 68-1 | Dabcyl | TCTACTACTGAATCA | OEK(ROX) |
| 58 | fluorescent probe HPV 68-2 | Dabcyl | TC2AC2ACTACAGA | OEK(ROX) |
| 59 | fluorescent probe HPV 68-3 | Dabcyl | TC2AC2ACTACAGA | OEK(ROX) |
| 60 | fluorescent probe HPV 70-1 | Dabcyl | CTGCTGTATATAGCC | OEK(CY5) |
| 61 | fluorescent probe HPV 70-2 | Dabcyl | ATAGCCCTACAAAGTT | OEK(CY5) |
| 62 | fluorescent probe HPV 70-3 | Dabcyl | CTGCTGTATTTAGCC | OK(ROX) |
| 63 | fluorescent probe HPV 82-1 | Dabcyl | TAACCATTAGCACT | OEK(CY5) |
| 64 | fluorescent probe HPV 82-2 | Dabcyl | CACTGCTGTTACTCCA | OEK(CY5) |
| 65 | fluorescent probe HPV 82-3 | Dab-K(Dab) | GCACTGCTGTTACTC | OEK(Cy5) |

| | | | | |
|---|---|---|---|---|
| (In the PNA sequences of Table 1, the numerical marks are where part of the PNA sequences is modified to D-glutamine and L-glutamine | | | | |

Each HPV genotype probe was designed in consideration of the sequence in HPV L1 region with high specificity for the genotype and the Tm value. In case where the Tm values of different genotypes overlapped, the fluorescent substance labeled on a PNA probe was altered for simultaneous identification of various genotypes in a tube. In addition, conformation of each probe was modified, and the number of mers was modified and the position thereof was adjusted to adjust the Tm value of fluorescent PNA.

**[Table 2]**

| PNA sequence modification | | | | | |
|---|---|---|---|---|---|
| Form | Base | Mark | Form | Base | Mark |
| D-glutamic acid | A | ① | L-glutamic acid | A | 1 |
| | T | ② | | T | 2 |
| | G | ③ | | G | 3 |
| | C | ④ | | c | 4 |

The PNA probes were synthesized by solid phase synthesis from PNA oligomers using PNA monomers protected by benzothiazolesulfonyl (Bts) and functionalized resins according to the method described in Korean Patent No. 464261 (Lee et al., Org. Lett., 9:3291, 2007). PNAs can be synthesized by other known method of 9-flourenylmethloxycarbonyl (Fmoc) or t-Boc(t-butoxycarbonyl) (Kim L. et al., J. Org. Chem., 59:5767, 1994; Stephen A. et al., Tetrahedron, 51:6179, 1995), and a report substance and a quenching substance were labeled on PNA probes according to methods widely used in the pertinent field.

### 1-2: Preparation of DNA oligomers

The PNA probes in Table were prepared by the inventors themselves and the various oligomers used for PCR reactions as in Table 3 were synthesized by Bionic (Korea). In particular, in order to improve HPV detection sensitivity and to identify the presence of absence of multiple infections with HPV genotypes by a real-time PCR reaction, primers that are allele-specific to the genotypes to be detected were designed and HPV genotype detection sensitivity and specificity depending on mixing of primers and fluorescent PNA probes were analyzed.

**[Table 3]**

| DNA oligomer sequences for analysis of characteristics of PNA probes | | |
|---|---|---|
| SEQ ID NO | Name | Sequence (5' → 3') |
| 66 | MY11 | GCMCAGGGWCATAAYAATGG |
| 67 | MY12 | GGACAGGGHCAYAAYAATGG |
| 68 | specific primer HPV 6-1 | TGCGTCATGTGGAAGAGTATGATTTAC |
| 69 | specific primer HPV 6-2 | TGTAAATCATACTCTTCCACATGACG |
| 70 | specific primer HPV 11-1 | GAATACATGCGCCATGTGGAGGA |
| 71 | specific primer HPV 11-2 | GCGCATGTATTCCTTATAATCTGA |
| 72 | specific primer HPV 16-1 | ACTAACTTTAARGAGTACCTACGACA |
| 73 | specific primer HPV 16-2 | CYTCCCCATGTCGTAGGTACTC |
| 74 | specific primer HPV 18-1 | CTACCAAATTTAAGCAGTATAGCAGA |
| 75 | specific primer HPV 18-2 | TCTGCTATACTGCTTAAATTTGGTAGCATC |
| 76 | specific primer HPV 26-1 | GACATGGCGAAGAATATGAATTACAATT |
| 77 | specific primer HPV 26-2 | TCTGTTGTAAGTGTTATTTTACACAACTG |
| 78 | specific primer HPV 33-1 | CATGTTGAAGAATATGATYTACAGTTTG |
| 79 | specific primer HPV 33-2 | CAAACTGTARATCATATTCTTCAACATGTC |
| 80 | specific primer HPV 45-1 | AGTAGACATGTGGAGGAATATGATTTACA |
| 81 | specific primer HPV 45-2 | CTCCACATGTCTACTATACTGC |
| 82 | specific primer HPV 52-1 | TACAATTTATTTTTCAATTGTGCAARA |
| 83 | specific primer HPV 52-2 | AGCYGTTAATGTAATYTTGCACAATTGA |
| 84 | specific primer HPV 58-1 | ATGTTGAAGARTATGACTTACAGTTTGTT |
| 85 | specific primer HPV 58-2 | AACAAACTGTAAGTCATAYTCTTCAACAT |
| 86 | specific primer HPV 69-1 | CAGTTTATAAGGCATGGTGAG |
| 87 | specific primer HPV 69-2 | TCCTCACCATGCCTTATAAACTG |
| 88 | specific primer HPV 73-1 | AGACATGCAGAAGAGTTTGATTTACAG |
| 89 | specific primer HPV 73-2 | TAGTAGAATTCATAGAATGTATATATGTCATTACCT |
| 90 | specific primer HPV 31-1 | GACATGGTGAGGAATTYGATTTACAATT |
| 91 | specific primer HPV 31-2 | CRAATTCCTCACCATGTCKTAAATAC |
| 92 | specific primer HPV 35-1 | CTTCTAAGTTTAAGGAATATACCAGGCAC |
| 93 | specific primer HPV 35-2 | CTTCACCATGCCTTAAATATTC |
| 94 | specific primer HPV 39-1 | GACATGTTGAAGAATATGATYTACAGTTTG |
| 95 | specific primer HPV 39-2 | CGTGCCTGGTATATTCCTTAAACTTAGA |
| 96 | specific primer HPV 51-1 | GAATATTTAAGGCATGGTGAAGAATATG |
| 97 | specific primer HPV 51-2 | CTAATATATTGCTTAAAGTTACTTGGAG |
| 98 | specific primer HPV 53-1 | CATGCAGAGGAATATGAATTACAATTTG |
| 99 | specific primer HPV 53-2 | ATTCCTCTGCATGYCTAACATACTG |
| 100 | specific primer HPV 56-1 | ATCAGTACCTTAGACATGTGGAGGARTATG |
| 101 | specific primer HPV 56-2 | CTCCACATGTCTAAGGTACTGAT |
| 102 | specific primer HPV 59-1 | CAGTTTTAAAGAATATGCCAGACATG |
| 103 | specific primer HPV 59-2 | GTCTGGCATATTCTTTAAAACTG |
| 104 | specific primer HPV 66-1 | TGAAATCAATCAATACCTTCGCCATGTG |
| 105 | specific primer HPV 66-2 | CATGGCGAAGGTATTGATTGATTTC |
| 106 | specific primer HPV 68-1 | TTAGGCATGTTGAGGAATATGATTTGC |
| 107 | specific primer HPV 68-2 | CAAATCATATTCCTCAACATGCCTAAC |
| 108 | specific primer HPV 70-1 | GCATGTGGAGGAATATGATTTACAA |
| 109 | specific primer HPV 70-2 | TCCACATGCCTAGTATATTCCT |
| 110 | specific primer HPV 82-1 | CAATTTATATTTCAATTGTGTAAAATCA |
| 111 | specific primer HPV 82-2 | TAGAATCCATGGTGTGCAGGTAA |

For identification of 11 HPV types in one reaction tube, the PNA fluorescent probes with SEQ ID NO: 1 to 32 in Table 1 were mixed at a proper ratio respectively with the primers with SEQ ID NO: 66 to 89 in Table 3. And then real-time PCR reactions were performed to identify the Tm value of each fluorescent probe by Melt measurement, and a combination of 11 types of probes that do not have overlapping fluorescent signals and the Tm values were selected. Using the combination of the 11 selected probes, a PCR reaction was carried out and detection sensitivity and specificity of HPV genotypes were analyzed. In addition, for identification of 11 HPV types in another reaction tube, the fluorescent PNA probes with SEQ ID NO: 33 to 65 in Table 1 were mixed at a proper ratio respectively with the primers with SEQ ID NO: 66, 67 and 90 to 111 in Table 3. And then real-time PCR reactions were performed to identify the Tm value of each fluorescent probe by Melt measurement, and a combination of 11 types of probes that do not have overlapping fluorescent signals and the Tm values were selected, followed by a PCR reaction and analysis of detection sensitivity and specificity of HPV genotypes.

### [Example 2]

### Analysis of reaction according to PNA probe mixtures and DNA oligomers

### 2-1: Preparation of the target nucleic acids

The DNAs used as the target nucleic acids were 22 genotypes of HPV for confirmation of specificity and sensitivity of combination of the probes and the primers. For each of the 22 genotypes of HPV, 10¹⁰ to 10⁰ copies of the DNA were prepared as the target nucleic acids.

### 2-2. Analysis of reaction of PNA probe mixtures and various primers

1) For identification of detection sensitivity and specificity of 11 HPV genotypes (6, 11, 16, 18, 26, 33, 45, 52, 58, 69 and 73) according to the target-specific PNA fluorescent probes prepared in Example 1-1 and the target-specific primer types prepared in Example 1-2, 0.15µM of each of the fluorescent PNA probes with SEQ ID NO: 1 to 32 in Table 1, 0.15µM of each of the primers with SEQ ID NO: 66 and 67 in Table 3, and 1.2µM of each of the primers with SEQ ID NO: 68 to 89 in Table 3 were mixed, to which PCR amplification solution (Enzynomics, Korea) and each of the prepared target nucleic acids were added. Then, a reaction was carried out using a real-time PCR machine (CFX96TM Real-time PCR System from Bio-Rad, USA). Forty five cycles were performed where a cycle involves reaction at 50°C for two minutes and 95°C for 15 minutes, followed by reaction at 95°C for 15 seconds, 55°C for 45 seconds, and 72°C for 15 seconds. Fluorescence of the PNA probes were measured at 55°C. Then, temperature was maintained at 95°C for five minutes before going down to 35°C. After that, the PNA probes and PCR reaction products were hybridized for five minutes, before elevating temperature by 0.5°C from 35°C to 80°C during which fluorescent signals were measured and the measured melting curves were analyzed.
2) In addition, for identification of detection sensitivity and specificity of 11 HPV genotypes (31, 35, 39, 51, 53, 56, 59, 66, 68, 70 and 82) according to the target-specific PNA fluorescent probes prepared in Example 1-1 and the target-specific primer types prepared in Example 1-2, 0.15µM of each of the fluorescent PNA probes with SEQ ID NO: 33 to 65 in Table 1, 0.15µM of each of the primers with SEQ ID NO: 66 and 67 in Table 3, and 1.2µM of each of the primers with SEQ ID NO: 90 to 111 in Table 3 were mixed, to which PCR amplification solution (Enzynomics, Korea) and each of the prepared target nucleic acids were added. Then, a reaction was carried out using a real-time PCR machine (CFX96TM Real-time PCR System from Bio-Rad, USA). Forty five cycles were performed where a cycle involves reaction at 50°C for two minutes and 95°C for 15 minutes, followed by reaction at 95°C for 15 seconds, 55°C for 45 seconds, and 72°C for 15 seconds. Fluorescence of the PNA probes were measured at 55°C.

Then, temperature was maintained at 95°C for five minutes before going down to 35°C. After that, the PNA probes and PCR reaction products were hybridized for five minutes, before elevating temperature by 0.5°C from 35°C to 80°C during which fluorescent signals were measured and the measured melting curves were analyzed.

### 2-3: Analysis of specificity and reaction of the selected PNA probe mixtures and the primers

For identification of detection sensitivity and specificity of 11 HPV genotypes (6, 11, 16, 18, 26, 33, 45, 52, 58, 69 and 73) identified in 2) of Example 2-2, 0.15µM of each of the mixture of the probe with SEQ ID NO: 35, 38, 41, 44, 47, 50, 53, 56, 59, 62 and 65 among the probes with SEQ ID NO: 33 to 65 in Table 1 and the primers with SEQ ID NO: 66 and 67 in Table 3, and 1.2µM of each of the primers with SEQ ID NO: 69, 71, 73, 75, 77, 79, 81, 83, 85, 87 and 89 were mixed, to which PCR amplification solution (Enzynomics, Korea) and each of the prepared target nucleic acids were added (Fig. 3).

In addition, for identification of detection sensitivity and specificity of the target-specific primer types and 11 HPV genotypes (31, 35, 39, 51, 53, 56, 59, 66, 68, 70 and 82) identified in 2) of Example 2-2, 1.2µM of each of the mixture of the probe with SEQ ID NO: 35, 38, 41, 44, 47, 50, 53, 56, 59, 62 and 65 among the probes with SEQ ID NO: 33 to 65 in Table 1 and the primers with SEQ ID NO: 66 and 67 in Table 3, and 1.2µM of each of the primers with SEQ ID NO: 91, 93, 95, 97, 99, 101, 103, 105, 107, 109 and 111 were mixed, to which PCR amplification solution (Enzynomics, Korea) and each of the prepared target nucleic acids were added. Then, a reaction was carried out using a real-time PCR machine (CFX96TM Real-time PCR System from Bio-Rad, USA). Forty five cycles were performed where a cycle involves reaction at 50°C for two minutes and 95°C for 15 minutes, followed by reaction at 95°C for 15 seconds, 55°C for 45 seconds, and 72°C for 15 seconds. Fluorescence of the PNA probes were measured at 55°C. Then, temperature was maintained at 95°C for five minutes before going down to 35°C. After that, the PNA probes and PCR reaction products were hybridized for five minutes, before elevating temperature by 0.5°C from 35°C to 80°C during which fluorescent signals were measured and the measured melting curves were analyzed.

As a result, it has been confirmed that, in the PCR reaction using the mixture of the fluorescent PNA probes specific to HPV genotypes and the primers, detection specificity of HPV genotypes is high and it is possible to identify HPV genotypes with excellent sensitivity.

### 2-4: Primers and fluorescence system for identifying HPV genotypes

For detection of other HPV genotypes that cannot be detected by the above-mentioned system of the mixture of the primers and probes, it has been confirmed, in a PCR reaction, whether various HPV genotypes can be detected, by designing forward primers MY11 and 12 and reverse primer GP6, which are well-known primer systems capable of detection of HPV genotype, and fluorescent probes to positions of sequences where the detection by using the SYBR green fluorescence detection system and the detection of various HPV genotypes are possible by using positions of sequences of PGMY09 as in Table 4.

**[Table 4]**

| Primer and probe system for detection of HPV genotypes | | |
|---|---|---|
| SEQ ID NO | Name | Sequence (5' → 3') |
| 112 | GP6 | TGATTTACAGTTTATTTTTC |
| 113 | MY09 | CGTCCMARRGGAWACTGATC |
| 114 | HPV detection PNA - 1 | TTTGTTACTGTGGTAGATAC |
| 115 | HPV detection PNA - 2 | TTTGTTACAGTTGTGGATAC |
| 116 | HPV detection PNA - 3 | TTTGTAACTGTTGTAGATAC |
| 117 | HPV detection PNA - 4 | TTTATTACCTGTGTTGATAC |
| 118 | HPV detection PNA - 5 | TTTGTAACTGTTGTGGATAC |
| 119 | HPV detection PNA - 6 | TTTTTAACTGTGGTGGACAC |
| 120 | HPV detection PNA - 7 | TTTATTACTTGTGTTGACAC |
| 121 | HPV detection PNA - 8 | TTTGTCACGGTGGTAGATAC |

As a result, it has been confirmed that the SYBR green detection system using the primers with SEQ ID NO: 66, 67, 112 and 113 is capable of detecting all of 18 HPV genotypes (types 30, 32, 34, 40, 42, 43, 44, 54, 55, 61, 62, 67, 74, 81, 83, 84, 87 and 90) other than the 22 types (types 6, 11, 16, 18, 26, 31, 33, 35, 39, 45, 51, 52, 53, 56, 58, 59, 66, 68, 69, 70, 73 and 82) by way of Ct values after the PCR reaction (Fig. 4). In addition, it has been confirmed that various HPV genotypes can be detected by using the fluorescent probe mixture of the probe detection systems 114 to 121. In the detection system using SYBR green, analysis on specificity was possible from positive and negative samples and, in case of false positive, analysis of the Tm value. In the detection system using the probe mixture, it was possible to identify between positive and negative samples with specificity since the HPV PCR reaction products amplified by the primers were linked with the specific fluorescent PNA probes.

### 2-5: Primer and fluorescent probe system for detecting HPV genotypes

it has been confirmed, in a PCR reaction, whether the 18 HPV genotypes (types 30, 32, 34, 40, 42, 43, 44, 54, 55, 61, 62, 67, 74, 81, 83, 84, 87 and 90) that cannot be detected in the above-mentioned system of the mixture of the primers and probes can be detected by forward primers MY11 and 12, which are primer systems that can detect the 18 HPV genotypes, and the fluorescent probes to positions of sequences where the detection using specific reverse primers that can amplify each of the 18 HPV genotypes, and the detection of the 18 HPV genotypes are possible, designed as in Tables 5 and 6.

**[Table 5]**

| SEQ ID NO | Name | Sequence (5' → 3') |
|---|---|---|
| 122 | specific primer HPV 55-1 | CAGGGCCACAATAATGG |
| 123 | specific primer HPV 55-2 | GCGCAGGGCCACAATAATGG |
| 124 | specific primer HPV 74-1 | GCGCAAGGCCACAAT |
| 125 | specific primer HPV 74-2 | GCGCAAGGCCACAATAATGG |
| 126 | specific primer HPV 87-1 | CCCAGGGCCACAATAAT |
| 127 | specific primer HPV 87-2 | GCCCAGGGCCACAATAATGG |
| 128 | specific primer HPV FHBB-1 | CATTTGCTTCTGACACAAC |
| 129 | specific primer HPV FHBB-2 | GCTTACATTTGCTTCTGACACAAC |
| 130 | specific primer HPV 30-1 | CTGTAATTCATATTCCTC |
| 131 | specific primer HPV 30-2 | CTGTAATTCATATTCCTCTACATGTC |
| 132 | specific primer HPV 42-1 | GCACATCATATTCTTCAGCA |
| 133 | specific primer HPV 42-2 | GCACATCATATTCTTCAGCATGTC |
| 134 | specific primer HPV 61-1 | AAATCAAACTCCTCTGTATG |
| 135 | specific primer HPV 61-2 | TGGCAAATCAAACTCCTCTGTATGGC |
| 136 | specific primer HPV 62-1 | AAATCAAATTCCTCCG |
| 137 | specific primer HPV 62-2 | GCAAATCAAATTCCTCCGTGTGTC |
| 138 | specific primer HPV 83-1 | TCATATTCCTCTGTGTG |
| 139 | specific primer HPV 83-2 | GTAAGTCATATTCCTCTGTGTGGCGG |
| 140 | specific primer HPV RHBB-1 | CATCCACGTTCACCTTGCC |
| 141 | specific primer HPV RHBB-2 | TTCATCCACGTTCACCTTGCC |
| 142 | specific primer HPV R1-1 | AYTGYAIKTCAWAYTCYTCIICAT |
| 143 | specific primer HPV R1-2 | AAAYTGYAIKTCAWAYTCYTCIICATG |
| 144 | specific primer HPV R2-1 | AYTGYAADTCAWAYTCYTCIIC |
| 145 | specific primer HPV R2-2 | AAAYTGYAADTCAWAYTCYTCIICATG |
| 146 | specific primer HPV R3-1 | TGYAAATCAWAYTCYTCIICATG |
| 147 | specific primer HPV R3-2 | AAAYTGYAAATCAWAYTCYTCIICATG |

**[Table 6]**

| SEQ ID NO | PNA name | PNA probe sequence | | |
|---|---|---|---|---|
| | | N-terminus | PNA sequence (N→C) | C-terminus |
| 148 | fluorescent probe HPV 30-1 | Dabcyl | CCACACAAACGTTATCCAC | OK(FAM) |
| 149 | fluorescent probe HPV 30-2 | Dabcyl | CCACACAAACGTTATCC | OK(FAM) |
| 150 | fluorescent probe HPV 32-1 | Dabcyl | TACTGTAACAACTGAAGAC | OK(FAM) |
| 151 | fluorescent probe HPV 32-1 | Dabcyl | CTACTGTAACAACTGAAG | OK(FAM) |
| 152 | fluorescent probe HPV 34-1 | Dabcyl | CAATCCACAAGTACAACTGC | OK(FAM) |
| 153 | fluorescent probe HPV 34-2 | Dabcyl | GTAGGTACACAATCCAC | OK(FAM) |
| 154 | fluorescent probe HPV 40-1 | Dabcyl | ACACCAACCCCATA | OK(FAM) |
| 155 | fluorescent probe HPV 40-2 | Dabcyl | CCACACCAACCCCATATA- | OK(FAM) |
| 156 | fluorescent probe HPV 42-1 | Dabcyl | TGCAACATCTGGTGATA | OK(FAM) |
| 157 | fluorescent probe HPV 42-2 | Dabcyl | CTGCAACATCTGGTGATAC | OK(FAM) |
| 158 | fluorescent probe HPV 43-1 | Dabcyl | CTCTACTGACCCTACTGTG | OK(FAM) |
| 159 | fluorescent probe HPV 43-2 | Dabcyl | CTCTACTGACCCTACTG | OK(FAM) |
| 160 | fluorescent probe HPV 44-1 | Dabcyl | CACAGTCCCCTCCGT | OK(FAM) |
| 161 | fluorescent probe HPV 44-2 | Dabcyl | TACACAGTCCCCTCCGT | OK(FAM) |
| 162 | fluorescent probe HPV 54-1 | Dabcyl | TCCACGCAGGATAGC | OK(FAM) |
| 163 | fluorescent probe HPV 54-2 | Dabcyl | CAGCATCCACGCAGGATAGC | OK(FAM) |
| 164 | fluorescent probe HPV 55-1 | Dabcyl | CAACTCAGTCTCCATC | OK(FAM) |
| 165 | fluorescent probe HPV 55-2 | Dabcyl | CAACTCAGTCTCCA | OK(FAM) |
| 166 | fluorescent probe HPV 61-1 | Dabcyl | CCCCCCTGTATCTGAA | OK(FAM) |
| 167 | fluorescent probe HPV 61-2 | Dabcyl | CCTGTATCTGAATA | OK(FAM) |
| 168 | fluorescent probe HPV 62-1 | Dabcyl | CTGCAGCAGAAT | OK(FAM) |
| 169 | fluorescent probe HPV 62-2 | Dabcyl | CGCCTCCACTGCTGCAGC | OK(FAM) |
| 170 | fluorescent probe HPV 67-1 | Dabcyl | ATCAGAGGCTAC | OK(FAM) |
| 171 | fluorescent probe HPV 67-2 | Dabcyl | TCAGAGGCTACATA | OK(FAM) |
| 172 | fluorescent probe HPV 74-1 | Dabcyl | CTCACAATCGCCTTCTGC | OK(FAM) |
| 173 | fluorescent probe HPV 74-2 | Dabcyl | CACAATCGCCTTCTG | OK(FAM) |
| 174 | fluorescent probe HPV 81-1 | Dabcyl | CAGCTACATCTGCTGC | OK(FAM) |
| 175 | fluorescent probe HPV 81-2 | Dabcyl | CAGCTACATCTGCT | OK(FAM) |
| 176 | fluorescent probe HPV 83-1 | Dabcyl | CAGCTGCTGCTACAC | OK(FAM) |
| 177 | fluorescent probe HPV 83-2 | Dabcyl | CAGCTGCTGCTAC | OK(FAM) |
| 178 | fluorescent probe HPV 84-1 | Dabcyl | CCACCACCGAATCAG | OK(FAM) |
| 179 | fluorescent probe HPV 84-2 | Dabcyl | CACCACCGAATCAGAA | OK(FAM) |
| 180 | fluorescent probe HPV 87-1 | Dabcyl | CACTCAAACAACCACTG | OK(FAM) |
| 181 | fluorescent probe HPV 87-2 | Dabcyl | CTCAAACAACCACTGA | OK(FAM) |
| 182 | fluorescent probe HPV 90-1 | Dabcyl | ACCCTCTGACACAT | OK(FAM) |
| 183 | fluorescent probe HPV 90-2 | Dabcyl | CCTCTGACACAT | OK(FAM) |
| 184 | fluorescent probe HBB-1 | Dab-K(Dab) | AGTCTGCCGTTACTGCC | OEK(Cy5) |
| 185 | fluorescent probe HBB-2 | Dab-K(Dab | GTCTGCCGTTACTGC | OEK(Cy5) |

As a result, it has been confirmed that the detection system using mixtures of the primers with SEQ ID NO: 66, 67 and 122 to 147 and the fluorescent probe of the probe detection systems 148 to 185 can detect all of 18 HPV genotypes by way of Ct values after the PCR reaction (Fig. 5). In the detection system using the fluorescent probe mixture, it was possible to identify between positive and negative samples with specificity since the HPV PCR reaction products amplified by the primers were linked with the specific fluorescent PNA probes.

The present application further includes the following additional preferred embodiments:
Preferred embodiment 1: A probe mixture comprising a PNA (Peptide Nucleic Acid) probe consisting of any one of nucleotide sequences of SEQ ID NO: 1 to 65, 114 to 121 and 148 to 185, wherein a reporter and a quencher are linked to the PNA probe, and the PNA probe is capable of genotype specifically binding to DNA of Human Papillomavirus (HPV).
Preferred embodiment 2: The probe mixture according to preferred embodiment 1, wherein the PNA probe detects the HPV genotype selected from the group consisting of types 6, 11, 16, 18, 26, 30, 31, 32, 33, 34, 35, 39, 40, 42, 43, 44, 45, 51, 52, 53, 54, 55, 56, 58, 59, 61, 62, 66, 67, 68, 69, 70, 73, 74, 81, 82, 83, 84, 87 and 90.
Preferred embodiment 3: The probe mixture according to preferred embodiment 1, wherein an amino acid, a side chain of an amino acid or a negatively charged molecule is linked to the PNA probe for structural modification.
Preferred embodiment 4: The probe mixture according to preferred embodiment 3, wherein the amino acid, the side chain of an amino acid or the negatively charged molecule is linked to N-terminus or C-terminus of the probe.
Preferred embodiment 5: The probe mixture according to preferred embodiment 3, wherein the amino acid, the side chain of an amino acid or the negatively charged molecule is linked to alpha, beta or gamma position of the probe backbone.
Preferred embodiment 6: The probe mixture according to preferred embodiment 1, wherein the reporter is at least one fluorescent substance selected from the group consisting of fluorescein, fluorescein chlorotriazinyl, rhodamine green, rhodamine red, tetramethylrhodamine, FITC, Oregon green, Alexa Fluor, FAM, JOE, ROX, HEX, Texas Red, CY5, CY3, TET, TRITC, TAMRA, a Cyanine-based dye and a thiadicarbocyanine dye.
Preferred embodiment 7: The probe mixture according to preferred embodiment 1, wherein the quencher is at least one selected from the group consisting of Dabcyl, TAMRA, Eclipse, DDQ, QSY, Blackberry Quencher, Black Hole Quencher, Qxl, Iowa black FQ, Iowa black RQ and IRDye QC-1.
Preferred embodiment 8: A method for detecting the HPV genotype, using one or more detection probes for real-time detection of a target nucleic acid,
   wherein the detection probe consists of any one of nucleotide sequences of SEQ ID NO: 1 to 65, 114 to 121 and 148 to 185, and wherein a reporter and a quencher are linked to the detection probe, and the detection probe is a PNA detection probe which is capable of genotype specifically binding to DNA of Human Papillomavirus (HPV).
Preferred embodiment 9: The method according to preferred embodiment 8, comprising:
   (a) performing hybridization by mixing a detection probe and a primer in the sample which contains the target nucleic acid to obtain a real-time amplification curve;
   (b) obtaining a melting curve between an amplified product and the detection probe with changing temperature after the amplification; and
   (c) analyzing the obtained real-time amplification curve and melting curve separately, sequentially or simultaneously.
Preferred embodiment 10: The method according to preferred embodiment 9, wherein the amplification is performed by a real-time polymerase chain reaction (PCR).

### INDUSTRIAL APPLICABILITY

The present invention can be applied to a method for detecting Human Papillomavirus (HPV) genotypes. In particular, the present invention relates to detection probes that are capable of genotype specifically binding to HPV DNA, a detection kit including them, and a method for detecting HPV genotypes using them. The present invention is capable of accurately detecting 22 HPV genotypes found in cervix, diagnosing coinfection with one or more HPV genotypes, detecting the presence or absence of other HPV genotypes than the 22 genotypes, and detecting HPV genotypes with high sensitivity and specificity.

## Claims

1. A probe mixture comprising
(i) a PNA (Peptide Nucleic Acid) probe consisting of nucleotide sequence of SEQ ID NO: 9 and
(ii) at least one PNA probe consisting of any one of nucleotide sequences of SEQ ID NO: 1 to 8, 10 to 65, 114 to 121 and 148 to 185,
wherein a reporter and a quencher are linked to the PNA probe, and the PNA probe is capable of genotype specifically binding to DNA of Human Papillomavirus (HPV).

2. The probe mixture according to claim 1, wherein the PNA probe detects the HPV genotype selected from the group consisting of types 6, 11, 16, 18, 26, 30, 31, 32, 33, 34, 35, 39, 40, 42, 43, 44, 45, 51, 52, 53, 54, 55, 56, 58, 59, 61, 62, 66, 67, 68, 69, 70, 73, 74, 81, 82, 83, 84, 87 and 90.

3. The probe mixture according to claim 1, wherein an amino acid, a side chain of an amino acid or a negatively charged molecule is linked to the PNA probe for structural modification.

4. The probe mixture according to claim 3, wherein the amino acid, the side chain of an amino acid or the negatively charged molecule is linked to N-terminus or C-terminus of the probe.

5. The probe mixture according to claim 3, wherein the amino acid, the side chain of an amino acid or the negatively charged molecule is linked to alpha, beta or gamma position of the probe backbone.

6. The probe mixture according to claim 1, wherein the reporter is at least one fluorescent substance selected from the group consisting of fluorescein, fluorescein chlorotriazinyl, rhodamine green, rhodamine red, tetramethylrhodamine, FITC, Oregon green, Alexa Fluor, FAM, JOE, ROX, HEX, Texas Red, CY5, CY3, TET, TRITC, TAMRA, a Cyanine-based dye and a thiadicarbocyanine dye.

7. The probe mixture according to claim 1, wherein the quencher is at least one selected from the group consisting of Dabcyl, TAMRA, Eclipse, DDQ, QSY, Blackberry Quencher, Black Hole Quencher, Qxl, Iowa black FQ, Iowa black RQ and IRDye QC-1.

8. A method for detecting the HPV genotype, using one or more detection probes for real-time detection of a target nucleic acid,
wherein the detection probes are
(i) a PNA (Peptide Nucleic Acid) probe consisting of nucleotide sequence of SEQ ID NO: 9 and
(ii) at least one PNA probe consistings of any one of nucleotide sequences of SEQ ID NO: 1 to 8, 10 to 65, 114 to 121 and 148 to 185,
and wherein a reporter and a quencher are linked to the detection probe, and the detection probe is a PNA detection probe which is capable of genotype specifically binding to DNA of Human Papillomavirus (HPV),
the method comprising the steps of:
(a) performing hybridization by mixing a detection probe and a primer in the sample which contains the target nucleic acid to obtain a real-time amplification curve;
(b) obtaining a melting curve between an amplified product and the detection probe with changing temperature after the amplification; and
(c) analyzing the obtained real-time amplification curve and melting curve separately, sequentially or simultaneously, wherein the amplification is performed by a real-time polymerase chain reaction (PCR).
